(19) Europäisches Patentamt
European Patent Office
Office européen des brevets

(11) Numéro de publication: **0 107 597 B1**

# (12) FASCICULE DE BREVET EUROPEEN

(45) Date de publication du fascicule du brevet: **26.02.86**

(51) Int. Cl.⁴: **C 07 K 5/06**, A 23 L 1/236

(21) Numéro de dépôt: **83420149.3**

(22) Date de dépôt: **15.09.83**

(54) **Composés chimiques et utilisation comme édulcorants.**

(30) Priorité: **17.09.82 FR 8215832**

(43) Date de publication de la demande:
**02.05.84 Bulletin 84/18**

(45) Mention de la délivrance du brevet:
**26.02.86 Bulletin 86/9**

(84) Etats contractants désignés:
**AT BE CH DE FR GB IT LI LU NL SE**

(56) Documents cités:
**EP-A-0 048 051**

(73) Titulaire: **UNIVERSITE CLAUDE BERNARD - LYON 1, 86 rue Pasteur, F-69007 Lyon (FR)**

(72) Inventeur: **Nofre, Claude, 119 Cours Albert Thomas, F-69003 Lyon (FR)**
Inventeur: **Tinti, Jean-Marie, 5 Avenue de Grenoble, F-69330 Meyzieu (FR)**

(74) Mandataire: **Laurent, Michel et al, 20 rue Louis Chirpaz Boîte Postale 32, F-69130 Lyon-Ecully (FR)**

EP 0 107 597 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

L'invention concerne un nouveau type de composés chimiques; elle concerne également des édulcorants de synthèse répondant à cette formule.

Comme on le sait, les "agents édulcorants", dénommés aussi "édulcorants", sont des composés capables de communiquer leur saveur sucrée aux divers produits alimentaires ou pharmaceutiques notamment, auxquels ils sont mélangés. L'utilisation d'édulcorants de synthèse peut être envisagée comme succédané du saccharose (sucre de table) dans les régimes hypoglucidiques des diabétiques et dans les régimes hypocaloriques des obèses du fait de leur faible apport calorique. Dans certains pays, l'utilisation d'édulcorants n'est pas limitée aux seuls besoins diététiques ou pharmaceutiques et leur utilisation dans l'alimentation courante a donné à ce type de composés une importance de tout premier ordre. Cependant, les composés les plus largement consommés à l'heure actuelle, à savoir le cyclamate de sodium et la saccharine, outre leur saveur parasite (arrière-goût amer pour la saccharine), sont remis en cause par suite de leurs possibles effets cancérogènes et leur utilisation a été, selon les pays, soit interdite, soit soumise à un contrôle.

Dans le brevet FR-A 1 577 545, correspondant aux brevets US-A 3492 131, 3714 139, 3642 491, 3800 046, on a décrit un édulcorant de synthèse connu sous le nom d'aspartam (dénomination commune internationale), dont le pouvoir sucrant se situe entre celui du cyclamate de sodium et celui de la saccharine. Ce produit toutefois présente l'inconvénient notable d'être cher, d'entraîner une surcharge de L-phénylalanine et d'être instable en se dégradant notamment en un dérivé dicétopipérazinique. Un des moyens possibles pour éviter cette cyclisation en dicétopipérazine est la substitution du groupe α-amino de l'acide L-aspartique.

Jusqu'à présent, peu de composés dérivés de l'aspartam et possédant le groupe α-amino substitué se sont révélés sucrés (voir par exemple EP-A-0048051). Cependant, ces composés ont un pouvoir sucrant identique à celui de l'aspartam et demeurent donc des produits chers, entraînant aussi une surcharge en L-phénylalanine.

L'invention pallie à ces inconvénients. Les composés selon l'invention présentent en effet l'avantage d'avoir le groupe α-amino de l'acide L-aspartique substitué et possèdent, à la différence des produits précédemment décrits, un pouvoir sucrant extrêmement élevé sans commune mesure avec celui de l'aspartam, ce qui est totalement inattendu. Par exemple, ce pouvoir sucrant pourra être jusqu'à 300 fois plus élevé que celui de l'aspartam, ce qui diminue considérablement le coût d'utilisation de cet agent édulcorant et réduit à une quantité négligeable la L-phénylalanine consommée.

Ces nouveaux composés chimiques se caractérisent en ce qu'ils comprennent un composé de formule générale I:

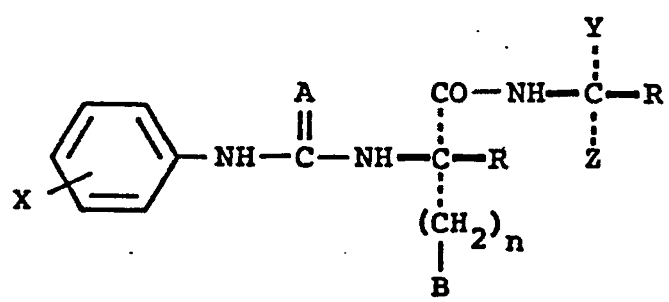

(I)

dans laquelle:

- X représente au moins un groupe donneur de doublets libres;
- A représente un atome d'oxygène ou de soufre, ou un groupe imino ou méthylène;
- R représente un atome d'hydrogène ou un groupe alkyle de 1 à 4 atomes de carbone;
- n représente un nombre égal à zéro ou à un;
- B représente un groupe COOH OU COOM (M désignant un cation);
- Y représente soit un groupe donneur de doublets libres, soit un groupe alkyle R' de 1 à 4 atomes de carbone;
- Z représente:

. soit un groupe hydrophobe R'', R'' désignant un groupe alkyle, cycloalkyle, benzyle ou aryle ayant jusqu'à douze atomes de carbone;

. soit un groupe hydrophobe R'', dans lequel 1 ou 2 atomes de carbone sont substitués par des atomes d'oxygène ou de soufre;

. soit un groupe hydrophobe R'' fixé par un groupe amidique, ester, un carbone halogéné ou alcoolique.

Comme on le sait, un "groupe donneur de doublets libres" est un groupe dont un atome au moins est porteur d'une ou plusieurs paire d'électrons non liés capables d'interagir par l'intermédiaire de liaisons hydrogène et un "groupe hydrophobe" est un groupe non polaire ou faiblement polaire.

Dans une forme d'application préférée, l'invention concerne plus particulièrement des agents édulcorants répondant à la formule générale (I) visée ci-dessus.

Avantageusement, dans les composés édulcorants selon l'invention:

- X est choisi dans l'ensemble constitué par les groupes CN, COOR, $SO_2R'$, COR', $NO_2$, halogènes, $SO_2NRR$, CONRR;
- A est choisi dans l'ensemble constitué par un atome d'oxygène ou de soufre;

- R est un atome d'hydrogène;
- n est égal à un;
- B est un groupe COOH ou COOM, M étant un cation choisi dans l'ensamble $Na^+$, $K^+$, $NH_4^+$, $Ca^{2+}$, $Mg^{2+}$, $Li^+$;
- Y est choisi dans l'ensemble constitué soit par les groupes COOR', $CH_2OR$, $CHOHCH_3$, $CONHCH_2CH_2OH$, $CONHCHRCONH_2$, $CF_3$, $CCl_3$, soit par un groupe R';
- Z est un groupe hydrophobe, choisi dans l'essemble constitué:

. soit par un groupe $CH_2C_6H_5$ (benzyl), $CH_2C_6H_{11}$ (cyclohexylméthyl), $C_6H_5$ (phényl), $C_6H_{11}$ (cyclohexyl), $C_5H_{11}$ (pentyl), iso-$C_5H_{11}$ (isopentyl), $C_4H_9$ (butyl), iso-$C_4H_9$ (isobutyl), ces groupes pouvant être diversement substitués, comme par exemple $CH_2C_6H_4OR$, $(CH_2)_xOR$, $(CH_2)_xSR'$, $(CH_2)_xCOOR'$, $(CH_2)_xSO_2$ R', $(CH_2)_xOCOR'$, avec x pouvant être égal à 0, 1 ou 2;

. soit par des amides (CONHR", CONR"R", CONHCHR"COOR', $CONHCHR''CONH_2$), des esters carboxyliques (COOR"), des dérivés halogénés (comme par exemple $CF_2R''$), des alcools (comme par exemple CHOHR").

En pratique:

- X est en position 4 et est choisi dans l'ensemble constitué par les groupes CN, $COOCH_3$, $COOC_2H_5$, $SO_2CH_3$, $COCH_3$;
- R est un atome d'hydrogène;
- n est égal à un;
- Y est choisi dans l'ensemble constitué par les groupes $COOCH_3$ ou $CH_3$;
- Z est choisi dans l'ensemble constitué par les groupes $CH_2C_6H_5$ (benzyl), $CH_2C_6H_{11}$ (cyclohexylméthyl), $C_6H_5$ (phényl), $C_6H_{11}$ (cyclohexyl), $C_5H_{11}$ (pentyl), iso-$C_5H_{11}$ (isopentyl), $CONHCH_2CH_2CH_3$ (propylamide), $CONHCH(CH_3)_2$ (isopropylamide), $CONHCH(C_3H_5)_2$ (dicyclopropylcarbinylamide), $CONHCH(C_3H_5)C(CH_3)_3$-(t-butylcyclopropylcarbinylamide),

Les composés édulcorants préférés de l'invention sont essentiellement:

- des dérivés du N-(4-X-phénylcarbamoyl ou 4-X-phénylthiocarbamoyl)-L-aspartyl-L-phénylalanine méthyl ester de formule générale suivante:

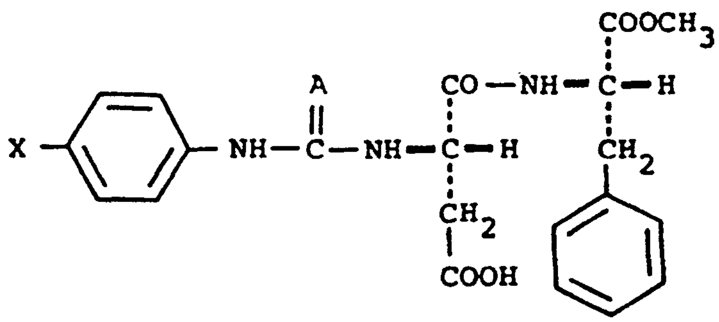

- des dérivés da la N-(4-X-phénylcarbamoyl ou 4-X-phénylthiocarbamoyl)-L-aspartyl-L-1-(1-Z)éthanamine:

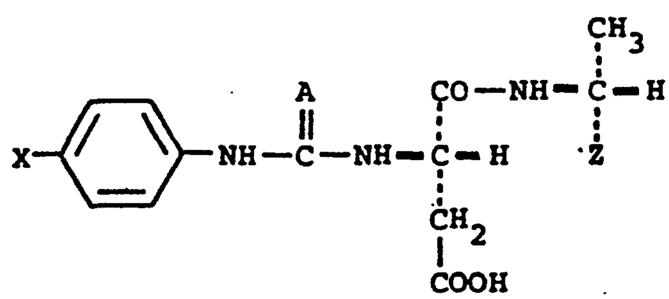

- des dérivés du N-(4-X-phénylcarbamoyl ou 4-X-phénylthiocarbamoyl)-L-aspartyl-D-alaninamide;

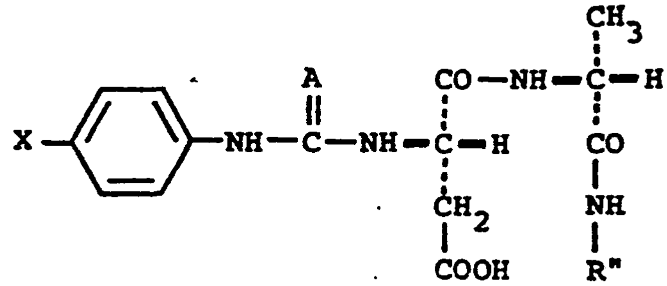

Les composés selon l'invention peuvent être préparés par condensation entre les composés de formules générales II et III:

$X—C_6H_4—N{=}C{=}A$ (II)

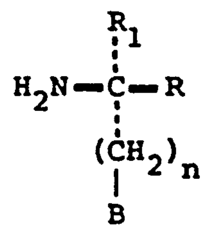

(III)

avec X, A, R, n et B correspondant aux définitions précédemment données et avec le groupe $R_1$ représentant soit un groupe

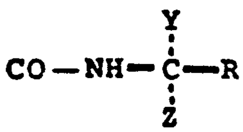

(avec Y et Z définis comme précédemment) soit un groupe COOH.

La réaction entre les composés de formules générales-II et III peut commodément être réalisée dans l'eau à température ambiante. Le composé II peut être préalablement dissous dans un solvant tel que le benzène, le chlorobenzène, le méthanol ou l'éthanol, ce qui permet d'améliorer notablement le rendement de la réaction.

Dans la cas ou $R_1$ est le groupe

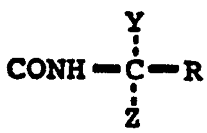

la composé III présente alors la formule générale suivante:

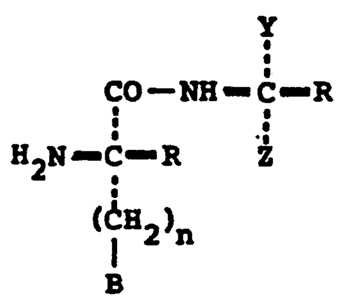

On obtient alors, aprés condensation avec le composé II, directemente le composé de formule générale I.

Dans le cas oû $R_1$ est le groupe COOH on met en contact le composé II avec le composé de formule générale IV:

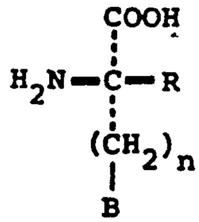

(IV)

dans lequel B peut être soit le groupe carboxyle libre, soit un groupe carboxyle préalablement protégé sous forme par exemple d'un ester benzylique ou tert-butylique. On obtient alors de la même façon le composé V de formule générale suivante:

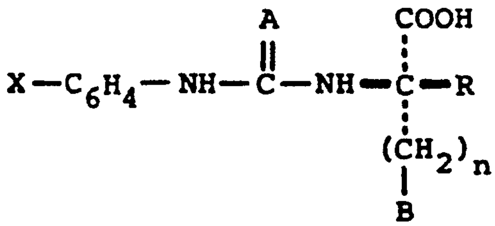

(V)

Ce composé est ensuite mis en contact avec le composé (VI) de formule générale:

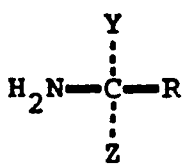

(VI)

dans laquelle Y, Z et R sont tels que définis précédemment. La réaction est effectuée en présence d'un agent déshydratant convenable, dont on peut citer plus particulièrement les alcoxyacétylènes, les carbodiimides avec notamment le dicyclohexylcarbodiimide, ou les sels d'isoxazolium. Cette réaction entre les composés V et VI peut aussi être réalisée en activant le groupe α-carboxyle libre ou le groupe amino des deux composés. L'activation du groupe carboxyle libre du composé V, qui est préférentiellement utilisée, est réalisée par diverses méthodes dont on peut signaler en particulier celles faisant intervenir la synthése d'un intermédiaire anhydride mixte, chlorure d'acide, azide ou d'un ester activé (comme par exemple un ester du p-nitrophénol ou du N-hydroxysuccinimide).

L'activation via un anhydride mixte formé in situ à partir d'un chloroformiate d'alkyle (tel que le chloroformiate d'éthyle ou de méthyle) est une des méthodes préférées. L'anhydride mixte est obtenu par addition de triéthylamine, puis de chloroformiate d'alkyle à une solution du composé V dans le tétrahydrofurane ou le diméthylformamide.

Cette réaction peut être effectuée entre - 20 °C et + 25 °C. L'anhydride mixte est rapidement formé (quelques minutes à - 10 °C) et le composé VI peut alors être ajouté. Après quelques heures, le composé I est directement obtenu après les traitements classiques, consistant en particulier en un lavage par des solutions acides et basiques.

Dans le cas particulier ou le groupe B du composé V est un groupe carboxyle protégé sous forme par exemple d'un ester benzylique ou tert-butylique, le déblocage de ce groupe protecteur est nécessaire pour obtenir le composé I avec le groupe carboxyle libre.

Ainsi, le groupe protecteur ester benzylique est éliminé par hydrogénolyse qui est la méthode de choix pour l'élimination de ce groupe. La réaction peut être réalisée commodément dans un solvant tel que le méthanol, l'éthanol ou l'acide acétique à 70 %, en présence de catalyseur comme le palladium sur charbon actif à 10 % sous légère pression d'hydrogène et à température ambiante. Dans le cas d'un groupe protecteur du type ester tert-butylique, son élimination est commodément réalisée par une hydrolyse acide qui peut être rapidement effectuée avec une solution de bromure d'hydrogène ou de chlorure d'hydrogène dans l'acide acétique glacial ou encore avec l'acide trifluoroacétique anhydre. On obtient finalement le composé I dans lequel le groupe B représente un groupe carboxyle libre.

Il est possible d'obtenir les composés de l'invention de façon différente notamment lorsque B est un groupe carboxyle libre et que n est égal à 1.

Dans ce cas, le diacide correspondant à la formule générale suivante:

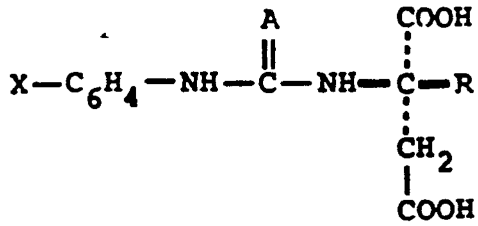

est transformé en anhydride de formule générale VII par action d'un agent de déshydratation convenable, comme par exemple l'anhydride acétique ou l'anhydride trifluoroacétique.

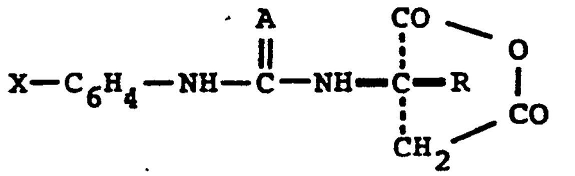

(VII)

Cette réaction peut commodément être réalisée en chauffant à reflux durant quelques minutes le diacide en présence de l'anhydride choisi. La présence d'un solvant tel que le benzène, le chloroforme ou le tétrahydrofurane ne s'avére pas indispensable pour cette réaction. Le composé VII ainsi obtenu est mis en présence du composé VI dissous dans un solvant inerte, tel que le diméthoxyéthane, le tétrahydrofurane ou l'acétate d'éthyle. Cette réaction conduit directement au composé I correspondant.

Les édulcorants selon l'invention peuvent être utilisés dans toutes les applications connues des agents édulcorants pour sucrer des substances consommables, solides ou liquides, par simple addition d'une quantité efficace de ces composés aux substances à consommer. Par "quantité efficace", on désigne une quantité qui soit détectable par les sensations physiologiques d'un être humain.

La manière dont l'invention peut être réalisée et les avantages qui en découlent ressortiront mieux des exemples de réalisation qui suivent donnés à titre indicatif et non limitatif.

**EXEMPLE 1:**

Synthèse du N-(4-cyanophénylcarbamoyl)-L-aspartyl-L-alanine méthyl ester (composé n° 12 du tableau I).

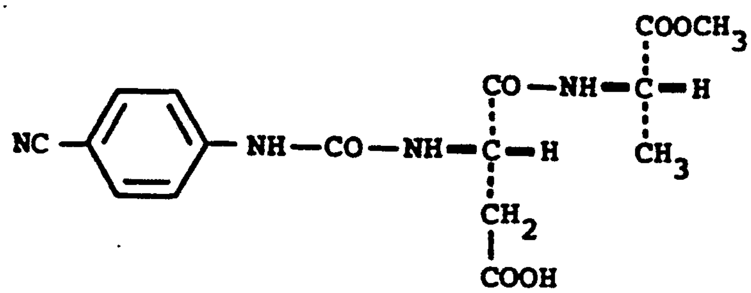

- **Etape 1**: Préparation de l'acide N-(4-cyanophénylcarbamoyl)-L-aspartique β-benzyl ester.

On dissout 3 g (13,4 mmol) d'acide L-aspartique β-benzyl ester dans 40 cm³ d'eau, puis on ajoute une solution concentrée de carbonate de sodium jusqu'à l'obtention d'un pH basique (≈ 9-10). On procéde alors à l'addition, sous forte agitation, de 2,2 g (15,2 mmol) de 4-cyanophényl isocyanate dissous dans 10 cm³ de benzène. La solution est vigoureusement agitée durant 2 heures à 20 °C avant filtration et extraction par 4 x 15 cm³ d'éther éthylique. La phase aqueuse est alors acidifiée jusqu'à pH = 1 par l'acide chlorhydrique puis extraite par 4 x 50 cm³ d'acétate d'éthyle. On obtient, après séchage sur sulfate de sodium anhydre et concentration, 4,5 g d'un produit huileux qui cristallise rapidement (rendement 93 %). Le solide a un point de fusion de 145-147 °C.

- **Etape 2**: Préparation du N-(4-cyanophénylcarbamoyl)-β-benzylester-L-aspartyl-L-alanine méthyl ester.

On dissout 1,2 g (3,3 mmol) de l'acide N-(4-cyanophénylcarbamoyl)-L-aspartique β-benzyl ester précédemment obtenu dans 20 $cm^3$ de diméthoxyéthane. La solution est refroidie à -10 °C avant l'addition de 0,33 g (3,3 mmol) de triéthylamine, puis de 0,36 g (3,3 mmol) de chloroformiate d'éthyle. On agite le mélange durant cinq minutes à la température de -10 °C avant l'addition de 0,41 g de L-alanine méthyl ester.

L'agitation est ensuite maintenue 5 minutes à -10 °C, puis 12 heures à la température ambiante. Après filtration et lavage par l'acétate d'éthyle du précipité formé, on concentre les filtrats à sec. Le résidu est repris dans l'acétate d'éthyle et la solution obtenue est lavée par 3 x 10 $cm^3$ d'acide chlorhydrique 2 N, par 3 x 10 $cm^3$ de carbonate de sodium à 10 %, puis par de l'eau (2 x 10 $cm^3$). Après séchage sur sulfate de sodium anhydre et concentration, on obtient 1,1 g d'un solide blanc (rendement 75 %) que l'on recristallise dans un mélange dichlorométhane/hexane. Le point de fusion du solide obtenu est de 185 °C.

- **Etape 3**: Préparation du N-(4-cyanophénylcarbamoyl)-L-aspartyl-L-alanine méthyl ester.

On dissout 0,65 g (1,4 mmol) du composé précédemment obtenu dans 30 $cm^3$ de méthanol en présence de 0,1 g de catalyseur au palladium sur charbon actif à 10 %. On soumet à une hydrogénation catalytique sous légère pression durant 4 heures à 20 °C. Le catalyseur est filtré et la solution est évaporée à sec.

On obtient un produit brut sous la forme d'une poudre blanche amorphe dont le point de fusion est de 129-130 °C. Ce composé possède une saveur sucrée équivalente à 400 (quatre cents) fois celle du saccharose.

**EXEMPLE 2** :

Synthése du N-(4-nitrophénylcarbamoyl)-L-aspartyl-L-norvaline méthyl ester (composé n°13 du tableau I).

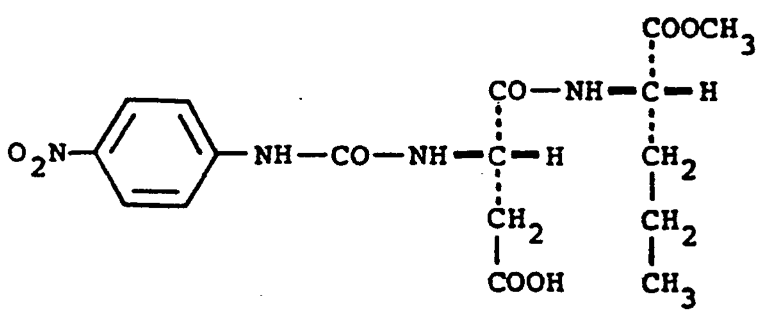

- **Etape 1:** Préparation de l'acide N-(4-nitrophényl-carbamoyl)-L-aspartique.

On dissout 4,56 g (0,0568 mol) d'acide L-aspartique dans l'eau par addition d'hydroxyde de sodium jusqu,à l'obtention d'un pH voisin de 10. On ajoute ensuite, à la température ambiante, une solution de 10 g (0,0568 mol) de 4-nitrophényl isocyanate dans 50 $cm^3$ de benzène. On agite vigoureusement durant deux heures tout en maintenant le pH autour de 10 par addition successive d'une solution d'hydroxyde de sodium concentrée. Le faible précipité formé est éliminé par filtration et le filtrat est lavé par 3 x 50 $cm^3$ d'éther éthylique. La phase aqueuse est alors acidifiée puis extraite par l'acétate d'éthyle, ce qui permet d'obtenir, après séchage sur sulfate de sodium anhydre et après concentration, 13,4 g (rendement 76 %) d'un solide jaune dont le point de fusion est de 118-120 °C.

- **Etape 2**: Préparation de l'anhydride de l'acideN-(4-nitrophénylcarbamoyl)-L-aspartique.

On verse 16 g (0,16 mol) d'anhydride acétique sur 20 g (0,067 mol) d'acide N-(4-nitrophénylcarbamoyl)-L-aspartique et on agite la pâte obtenue 10 minutes à 20 °C, puis 30 minutes à reflux. On diminue le volume de la solution obtenue par concentration sous vide et on ajoute de l'éther éthylique anhydre qui fait précipiter l'anhydride formé. On obtient ainsi 18 g d'un solide (rendement 95 %) dont le point de fusion est de 202-204 °C.

- **Etape 3**: Préparation du N-(4-nitrophénylcarbamoyl)-L-aspartyl-L-norvaline méthyl ester.

On dissout 1,05 g (3,6 mmol) d'anhydride de l'acide N-(4-nitrophénylcarbamoyl)-L-aspartique dans 20 $cm^3$ d'acétate d'éthyle, puis on ajoute 0,47 g (3,6 mmol) de L-norvaline méthyl ester. La solution est ensuite maintenue trois jours à 30 °C. On lave le mélange réactionnel par 3 x 10 $cm^3$ d'acide chlorhydrique N puis on le sèche sur sulfate de sodium anhydre avant concentration à sec. Le produit obtenu est purifié par dissolution en milieu basique suivie d'une précipitation par une solution d'acide chlorhydrique N jusqu'à obtention d'un pH de 4,5. On obtient 0,6 g d'un produit brut (rendement 35 %) qui se présente sous la forme d'une poudre amorphe dont le point de fusion est de 220-222 °C. Ce composé possède une saveur sucrée équivalente à 2500 (deux mille cinq cents) fois celle du saccharose.

**EXEMPLE 3** :

Synthèse du N-(4-nitrophénylcarbamoyl)-L-aspartyl-L-norleucine méthyl ester (composé n°14 du tableau I).

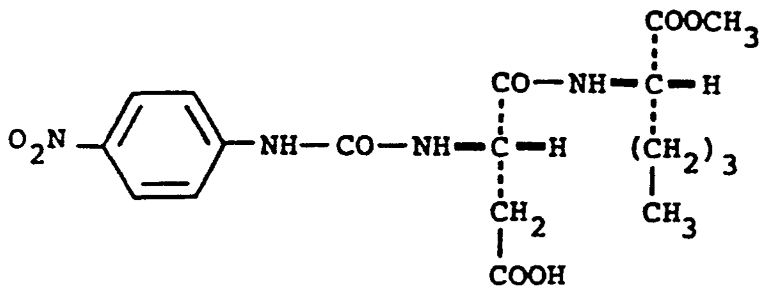

- **Etape 1**: Préparation du N-(benzyloxycarbonyl)-β-benzylester-L-aspartyl-L-norleucine méthyl ester.

A partir de 3 g (8,4 mmol) d'acide N-(benzyloxycarbonyl)-β-benzylester-L-aspartique et de 1,35 g (8,4 mmol) de L-norleucine méthyl ester, on obtient suivant le protocole de l'exemple 1 (étape 2), 3,2 g (rendement 80 %) d'un solide dont le point de fusion est de 85 °C.

- **Etape 2**: Préparation du L-aspartyl-L-norleucineméthyl ester.

On dissout le composé précédemment obtenu dans 30 $cm^3$ de méthanol en présence de 0,2 g de catalyseur au palladium sur charbon actif à 10 % et on soumet à l'hydrogénation catalytique sous légère pression durant 4 heures à 20 °C. Après filtration de la solution et concentration, on obtient 1,65 g d'un solide blanc après trituration dans l'éther éthylique.

**Etape 3** : Préparation du N-(4-nitrophénylcarbamoyl)-L-aspartyl-L-norleucine méthyl ester.

On dissout 0,51 g (1,9 mmol) de L-aspartyl-L-norleucine méthyl ester dans 20 $cm^3$ d'eau et on ajoute quelques $cm^3$ d'une solution concentrée de carbonate de sodium pour amener le pH de la solution à 9-10. On ajoute à la température ambiante et sous très forte agitation 0,6 g (3,8 mmol) de 4-nitrophényl isocyanate dissous dans 5 $cm^3$ de benzène. Après deux heures de contact, on filtre le précipité formé et on lave le filtrat aqueux par 3 x 15 $cm^3$ d'éther éthylique avant de diminuer sous vide son volume de moitié. On acidifie par une solution d'acide chlorhydrique N jusqu'à obtention d'un pH de 4,5, puis on filtre et on lave par quelques $cm^3$ d'eau le précipité formé. On obtient 0,7 g d'un produit brut (rendement 84 %) qui se présente sous la forme d'une poudre amorphe dont le point de fusion est de 204-205 °C.

Ce composé possède une intense saveur sucrée équivalente à 13 000-14 000 (treize à quatorze mille) fois celle du saccharose.

**EXEMPLE 4:**

Synthèse du N-(4-cyanophénylcarbamoyl)-L-aspartyl-L-phénylalanine méthyl ester (composé n° 6 du tableau I).

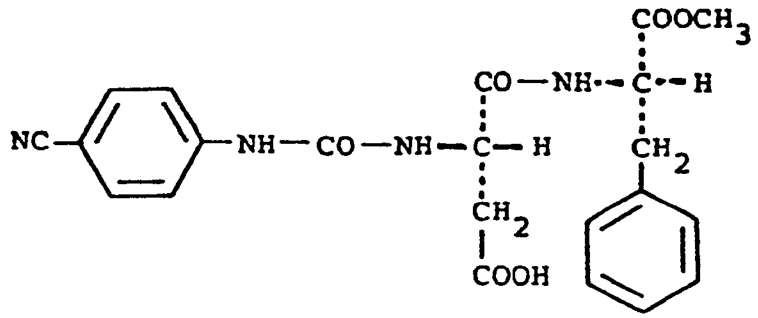

A une solution de 25 cm$^3$ d'eau contenant 1 g (3,39 mmol) de L-aspartyl-L-phénylalanine méthyl ester et 0,21 g (1,98 mmol) de carbonate de sodium anhydre, est ajoutée une solution de 0,49 g (3,40 mmol) de 4-cyanophényl isocyanate dissous dans 10 cm$^3$ de benzène.

Une agitation vigoureuse est maintenue 15 minutes à température ambiante avant de procéder à l'extraction du melange par 3 x 20 cm$^3$ d'acétate d'éthyle. La phase aqueuse est refroidie puis acidifiée lentement par une solution d'acide chlorhydrique N jusqu'à l'obtention d'un pH d'environ 4,5. Le précipité formé est filtré, lavé par trois fractions de 5 cm$^3$ d'eau froide, puis séché sous vide en présence de diphosphore pentaoxyde. On obtient 1,31 g d'un produit brut (rendement 88 %) qui se présente sous la forme d'une poudre amorphe dont le point de fusion est de 179-181 °C. Après recristallisation dans l'alcool éthylique à 95 %, on obtient un solide cristallin dont le point de fusion est de 210 °C. Ce composé possède une intense saveur sucrée équivalente à 10 000 (dix mille) fois celle du saccharose.

**EXEMPLE 5:**

Synthèse du N-(4-cyanophénylthiocarbamoyl)- L-aspartyl-L- phénylalanine méthyl ester (composé 10 du tableau I).

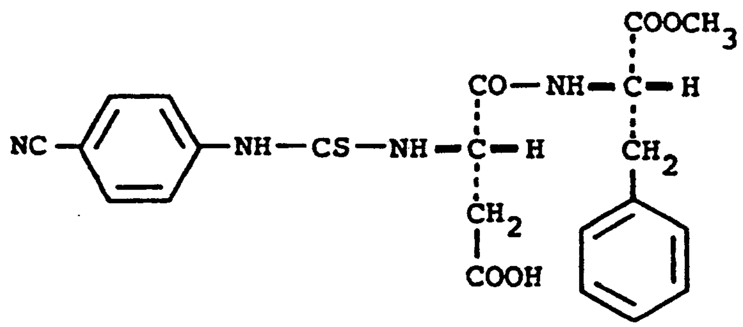

On verse goutte à goutte une solution de 6 g (0,051 mol) de 4-cyanoaniline dans 50 cm$^3$ d'acide chlorhydrique N sur une suspension de 6,5 g (0,056 mol) de thiophosgène dans 100 cm$^3$ d'eau. Le milieu réactionnel est vigoureusement agité durant deux heures à température ambiante. Le précipité formé est filtré, lavé par trois fractions de 10 cm$^3$ d'eau, puis séché sous vide, en présence de diphosphore pentaoxyde.

On recristallise dans un mélange d'hexane et de tétrachlorure de carbone. On obtient 7,2 g de 4-cyanophényl isothiocyanate (rendement 85 %) dont le point de fusion est de 117-118 °C.

A une solution de 25 cm$^3$ d'eau contenant 1 g (3,39 mmol) de L-aspartyl-L-phénylalanine méthyl ester et 0,21 g (1,98 mmol) de carbonate de sodium anhydre est ajouté 0,55 g (3,40 mmol) de 4-cyanophényl isothiocyanate dissous dans 10 cm$^3$ d'éthanol. La solution est vigoureusement agitée durant deux heures à température ambiante. Après concentration par évaporation sous vide, on effectue un lavage de la solution aqueuse par trois fois 20 cm$^3$ d'acétate d'éthyle.

Après refroidissement, on acidifie lentement par l'acide chlorhydrique N jusqu'à l'obtention d'un pH d'environ 4,5. Le précipité est filtré, lavé par trois fractions de 5 cm$^3$ d'eau froide, puis séché sous vide en présence de diphosphore pentaoxyde. On obtient 1 g d'un produit brut (rendement 65 %) qui se présente sous la forme d'une poudre amorphe dont le point de fusion est de 172-173 °C. Ce composé possède une très intense saveur sucrée équivalente à 50 000 (cinquante mille) fois celle du saccharose.

Comme on l'a constaté, le pouvoir édulcorant des composés de synthèse conformes à l'invention peut être de l'ordre de dix mille à cinquante mille, c'est-à-dire de cinquante à trois cents fois celui des composés actuellement commercialisés les plus performants.

Ce pouvoir édulcorant est apprécié de la manière suivante.

**EVALUATION DU POUVOIR SUCRANT.**

Les solutions aqueuses des composés ont été goûtées par un jury entraîné et comparées à des solutions témoins de saccharose. Ces solutions témoins ont été choisies à des concentrations comprises entre 2 et 10 %, c'est-à-dire à des concentrations correspondant à celles utilisées lors d'un usage courant. Cependant une solution témoin à 2 % (0,058 mol/l) de saccharose a été utilisée préférentiellement pour toutes les évaluations sensorielles; à cette concentration, la saveur sucrée de la solution reste bien perceptible par l'ensemble des dégustateurs et permet des comparaisons plus précises que celles obtenues avec des solutions de saccharose de concentrations plus élevées.

Le pouvoir sucrant est évalué en effectuant le rapport molaire ou pondéral, à l'isosucrosité (c'est-à-dire à saveurs sucrées identiques), entre la solution de saccharose témoin et la solution du composé.

Cette propriété des composés de l'invention de pouvoir communiquer leur intense saveur sucrée aux produits auxquels ils sont associés peut être illustrée par les exemples suivants.

**Essai 1**:

On prépare une solution de 0,5 milligramme du N-(4-nitrophénylthiocarbamoyl)-L-aspartyl-L-phénylalanine méthyl ester (composé 11 du tableau I) dans un litre d'eau. Lors des essais organoleptiques, on constate que cette solution présente une saveur sucrée équivalente à celle d'une solution de saccharose à 2 %, ce qui correspond à un pouvoir sucrant de 55 000 fois celui du saccharose sur une base molaire (40 000 sur une base pondérale). On observe que ce même composé, en comparaison avec des solutions témoins de saccharose à 5 % et 10 %, possède un pouvoir sucrant respectivement de 38 000 et 23 000 (soit 28 500 et 17 000 sur une base pondérale). Le pouvoir sucrant du composé comparé à une solution à 1 % de saccharose (correspondant à une concentration un peu supérieure à celle du seuil de perception de la saveur sucrée) est de 70 000 sur une base molaire et de 52 500 sur une base pondérale.

**Essai 2**:

On prépare un thé, par infusion de feuilles de thé, ou un café, par dissolution de 26 g de café instantané dans un litre d'eau. On constate que 100 $cm^3$ de ces solutions, sucrées avec 0,5 milligramme du N-(4-cyano-phénylcarbamoyl)-L-aspartyl-L-phénylalanine méthyl ester (composé 6 du tableau I), ne peuvent être différenciées des mêmes solutions de thé ou de café contenant 5 g de saccharose. Le pouvoir sucrant du composé est dans ce cas de 12 800 sur une base molaire comparativement à une solution de saccharose à 5 % (soit 10 000 sur une base pondérale). On observe le même effet en remplaçant le saccharose par 20 mg d'aspartam. Dans ce cas le composé est 60 fois plus sucré que l'aspartam sur une base molaire et 40 fois sur une base pondérale.

**Essai 3**:

Le pouvoir sucrant de quelques composés de l'invention répondant à la formule générale (I) a été évalué comparativement au saccharose à 2 %; les valeurs sont données en valeur relative sur une base molaire dans le tableau I, dans lequel X, A, B, Y, Z désignent les radicaux de la formule générale I et F (°C) le point de fusion du produit brut obtenu directement par précipitation dans l'eau; pour les composés de ce tableau, le radical R de la formule générale est toujours un atome d'hydrogène et la valeur de n est toujours égale à 1.

TABLEAU I

| Composé n° | X en position 4 | A | B | Y | Z | F (°C) | Pouvoir sucrant (saccharose = 1) |
|---|---|---|---|---|---|---|---|
| 1 | F | O | COOH | $COOCH_3$ | $CH_2C_6H_5$ | 183-184 | 500 |
| 2 | $CH_3CO$ | O | COOH | $COOCH_3$ | $CH_2C_6H_5$ | 156-158 | 2 200 |
| 3 | $CH_3OCO$ | O | COOH | $COOCH_3$ | $CH_2C_6H_5$ | 183-184 | 3 800 |
| 4 | $C_2H_5OCO$ | O | COOH | $COOCH_3$ | $CH_2C_6H_5$ | 188-189 | 2 000 |
| 5 | $CH_3SO_2$ | O | COOH | $COOCH_3$ | $CH_2C_6H_5$ | 133-135 | 2 100 |
| 6 | NC | O | COOH | $COOCH_3$ | $CH_2C_6H_5$ | 179-181 | 10 000 |
| 7 | $O_2N$ | O | COOH | $COOCH_3$ | $CH_2C_6H_5$ | 196-198 | 14 000 |
| 8 | $CH_3OCO$ | S | COOH | $COOCH_3$ | $CH_2C_6H_5$ | 93-94 | 27 000 |
| 9 | $C_2H_5OCO$ | S | COOH | $COOCH_3$ | $CH_2C_6H_5$ | 99-100 | 13 000 |
| 10 | NC | S | COOH | $COOCH_3$ | $CH_2C_6H_5$ | 172-173 | 50 000 |
| 11 | $O_2N$ | S | COOH | $COOCH_3$ | $CH_2C_6H_5$ | 146-147 | 55 000 |
| 12 | NC | O | COOH | $COOCH_3$ | $CH_3$ | 129-130 | 400 |
| 13 | $O_2N$ | O | COOH | $COOCH_3$ | $C_3H_7$ | 220-222 | 2 500 |
| 14 | $O_2N$ | O | COOH | $COOCH_3$ | $C_4H_9$ | 204-205 | 14 000 |
| 15 | NC | O | COOH | $COOCH_3$ | $C_6H_5$ | 194-197 | 10 000 |
| 16 | $O_2N$ | O | COOH | $COOCH_3$ | $C_6H_{11}$ | 203-205 | 8 000 |

**TABLEAU I** (suite)

| Composé n° | X en position 4 | A | B | Y | Z | F (°C) | Pouvoir sucrant (saccharose = 1) |
|---|---|---|---|---|---|---|---|
| 17 | $O_2N$ | O | COOH | $COOCH_3$ | $CH_2C_6H_{11}$ | 178-180 | 5 500 |
| 18 | $O_2N$ | O | COOH | $COOCH_3$ | $CH_2OC(CH_3)_3$ | 196-198 | 3 500 |
| 19 | $O_2N$ | O | COOH | $COOCH_3$ | $COOCH_3$ | 202-203 | 13 000 |
| 20 | NC | O | COOH | $COOCH_3$ | $CF_3$ | 196-197 | 2 500 |
| 21 | $O_2N$ | O | COOH | $COOC_2H_5$ | $COOC_2H_5$ | 181-182 | 15 000 |
| 22 | $O_2N$ | O | COOH | $CH_3$ | $COOCH_3$ | 175-177 | 4 500 |
| 23 | $O_2N$ | O | COOH | $CH_3$ | $COOC_2H_5$ | 190-193 | 7 000 |
| 24 | $O_2N$ | O | COOH | $CH_3$ | $COOC_3H_7$ | 174-177 | 9 000 |
| 25 | $O_2N$ | O | COOH | $CH_3$ | $CONHC_3H_7$ | 204-207 | 300 |
| 26 | NC | O | COOH | $CH_3$ | $C_6H_5$ | 204-206 | 1 700 |
| 27 | NC | S | COOH | $CH_3$ | $C_6H_5$ | 88-90 | 3 500 |
| 28 | NC | O | COOH | $CH_3$ | $(CH_2)_2CH(CH_3)_2$ | 173-175 | 2 800 |
| 29 | NC | S | COOH | $CH_3$ | $(CH_2)_2CH(CH_3)_2$ | 125-126 | 9 000 |
| 30 | $O_2N$ | O | COOH | $CH_2OH$ | $COOCH_3$ | 175-179 | 1 700 |
| 31 | NC | O | COOH | $CH_2OH$ | $COOC_3H_7$ | 170-172 | 2 500 |
| 32 | NC | O | COOH | $CH_2OH$ | $COOCH(CH_3)_2$ | 173-175 | 1 700 |

A titre indicatif, les pouvoirs sucrants de quelques produits connus ou commercialisés à ce jour, mesurés par la même méthode, sont donnés dans le tableau II.

TABLEAU II

| Composé | Pouvoir sucrant (comparé au saccharose entre 2 et 10 %) |
|---|---|
| Xylitol | 1,1 |
| Cyclamate de sodium | 30-50 |
| Glycyrrhizine | 50 |
| Acésulfam-K | 100-130 |
| Aspartam | 100-200 |
| Saccharine | 200-400 |

Comme déjà dit, les composés selon l'invention possèdent une saveur sucrée agréable comparable à celle du saccharose.

Le pouvoir sucrant de ces composés varie, comme pour tous les composés édulcorants, suivant la concentration de la solution de saccharose utilisée comme référence. Par exemple, le composé 11 du tableau I possède un pouvoir sucrant de 55 000 (cinquante cinq mille) par rapport à une solution de saccharose à 2 %, de 38 000 (trente huit mille) par rapport à une solution de saccharose à 5 %, et de 23 000 (vingt trois mille) par rapport à une solution de saccharose à 10 %. Les concentrations de saccharose comprises entre 2 et 10 % sont celles fréquemment utilisées dans la vie courante.

Le pouvoir sucrant de ces composés varie aussi, comme pour les autres édulcorants, suivant la nature du produit à édulcorer.

Généralement, les composés conformes à l'invention se présentent sous forme solide et, dans cet état, ils sont stables pendant plusieurs mois dans les conditions usuelles de conservation.

En solution aqueuse à 25 °C et à pH = 4-7, la stabilité de ces produits est compatible avec les applications envisagées.

La solubilité de ces composés dans l'eau est moyenne, mais elle s'avère suffisante étant donné la très faible quantité de produit nécessaire pour obtenir un fort pouvoir sucrant. La solubilité de ces composés peut être nettement améliorée en les utilisant sous forme de sel, tel que par exemple sous forme de sel de sodium ou de potassium.

Par ailleurs, les nouveaux édulcorants de synthèse selon l'invention présentent de nombreux avantages par rapport aux produits commercialisés actuellement, notamment par rapport à l'aspartam. On peut citer:

- un pouvoir sucrant de 40 à 300 fois en moyenne supérieur à l'aspartam, ce qui est surprenant et était totalement imprévisible;
- d'excellentes propriétés organoleptiques;
- une toxicité qui peut être compatible avec les applications envisagées, puisque les produits selon l'invention doivent être utilisés à des concentrations en moyenne quarante à trois cents fois plus faibles que l'aspartam;
- du fait des faibles concentrations ainsi utilisées, les risques secondaires dûs à une consommation excessive d'amino-acides, et notamment de L-phénylalanine (qui présente un inconvénient pour les personnes atteintes de phénylcétonurie), sont très réduits;
- du fait également des faibles concentrations ainsi utilisées, ces produits présentent un apport calorique négligeable, d'où leur possibilité d'emploi dans divers régimes alimentaires;
- par suite de l'absence d'un groupe amino libre, les composés selon l'invention ne présentent pas l'inconvénient d'une instabilité due à une cyclisation intramoléculaire en dérivé dicétopipérazinique, comme cela est le cas pour l'aspartam;
- les composés de l'invention présentent un avantage économique important dû au fait que de faibles quantités de produit sont nécessaires pour édulcorer une préparation, ce qui diminue d'autant leur coût d'utilisation;
- ces produits sont non cariogènes, d'ou leur avantage pour leur utilisation dans les pâtes dentifrices et les pâtes à mâcher.

De la sorte, on peut les utiliser avec succès comme produits édulcorants, notamment dans les régimes hypoglucidiques des diabétiques, dans les régimes hypocaloriques des obèses ou dans les régimes basse calorie des pléthoriques, ainsi que pour toutes applications ou l'on recherche un goût sucré. A titre d'exemples, on peut citer les aliments (comme par exemple les pâtisseries, les plats cuisinés, les confitures, les crèmes, les glaces, les produits à base de lait, les fruits), les boissons (comme par exemple les jus de fruits, les jus de légumes, les sirops, les boissons gazeuses, les boissons instantanées en poudre, le café, le thé, le lait), les confiseries, les chewing-gums. Les composés selon l'invention peuvent également avoir pour intérêt de pouvoir améliorer la saveur de certains produits de toilette, cosmétiques et articles d'hygiène (comme par exemple les dentifrices,

les bains de bouche, les gargarismes, les rouges à lèvres, les masticatoires), de certaines préparations à usage pharmaceutique ou vétérinaire (pour améliorer le goût de la préparation ou pour masquer le goût déplaisant de certaines drogues), et les aliments pour animaux.

**Revendications** pour les Etats contractants BE, CH, DE, FR, GB, IT, LI, LU, NL, SE

Composés chimiques, caractérisés en ce qu'ils comprennent un composé de formule générale:

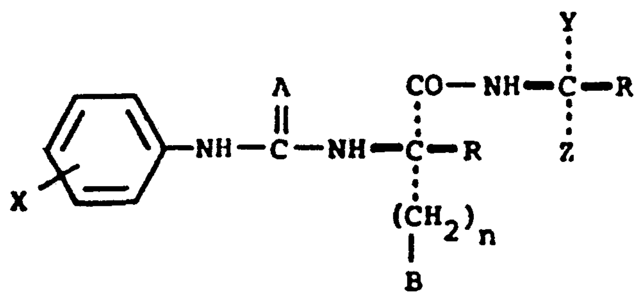

dans laquelle:
- X représente au moins un groupe donneur de doublets libres;
- A représente un atome d'oxygène ou de soufre ou un groupe imino ou méthylène;
-R représente un atome d'hydrogène ou un groupe alkyle de 1 à 4 atomes de carbone;
- n représente un nombre égal à zéro ou à un;
- B représente un groupe COOH ou COOM (M désignant un cation);
- Y représente soit un groupe donneur de doublets libres, soit un groupe alkyle R' de 1 à 4 atomes de carbone;
- Z représente:

. soit un groupe hydrophobe R'', R'' désignant un groupe alkyle, cycloalkyle, benzyle ou aryle, ayant jusqu'à douze atomes de carbone;

. soit un groupe hydrophobe R'' dans lequel un ou deux atomes de carbone sont substitués par des atomes d'oxygène ou de soufre;

. soit un groupe hydrophobe R'' fixé par un groupe amidique, ester, un carbone halogéné ou alcoolique.

2. Edulcorants de synthèse, caractérisés en ce qu'ils comprennent un composé de formule générale:

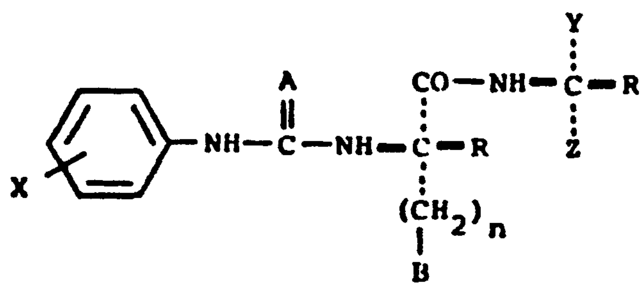

dans laquelle:
- X représente au moins un groupe donneur de doublets libres;
- A représente un atome d'oxygène ou de soufre ou un groupe imino ou méthylène;
- R représente un atome d'hydrogène ou un groupe alkyle de 1 à 4 atomes de carbone;
- n représente un nombre égal à zéro ou à un;
- B représente un groupe COOH ou COOM (M désignant un cation);
- Y représente soit un groupe donneur de doublets libres soit un groupe alkyle R' de 1 à 4 atomes de carbone;
- Z représente:

. soit un groupe hydrophobe R'', R'' designant un groupe alkyle, cycloalkyle, benzyle ou aryle ayant jusqu'à douze atomes de carbone;

. soit un groupe hydrophobe R'', dans lequel 1 ou 2 atomes de carbone sont substitués par des atomes d'oxygène ou de soufre;

. soit un groupe hydrophobe R'' fixé par un groupe amidique, ester, un carbone halogéné ou alcoolique.

3. Edulcorants selon la revendication 2, caractérisé en ce que:
- X est choisi dans l'ensemble constitué par les groupes CN, COOR, $SO_2R'$, COR', $NO_2$, halogènes, $SO_2NRR$, CONRR.
- A est choisi dans l'ensemble constitué par un atome d'oxygène ou de soufre;
- R est un atome d'hydrogène;

- n est égal à un;
- B est un groupe COOH ou COOM, M étant un cation choisi dans l'ensemble $Na^+$, $K^+$, $NH_4^+$, $Ca^{2+}$, $Mg^{2+}$, $Li^+$;
- Y est choisi dans l'ensemble constitué soit par les groupes COOR', $CH_2OR$, $CHOHCH_3$, $CONHCH_2CH_2OH$, $CONHCHRCONH_2$, $CF_3$, $CCl_3$, soit un groupe R';
- Z est choisi dans l'ensemble constitué:
. soit par un groupe $CH_2C_6H_5$ (benzyl), $CH_2C_6H_{11}$ (cyclohexylméthyl), $C_6H_5$ (phényl), $C_6H_{11}$ (cyclohexyl), $C_5H_{11}$ (pentyl), iso-$C_5H_{11}$ (isopentyl), $C_4H_9$ (butyl), iso-$C_4H_9$ (isobutyl),
. soit par un de ces groupes substitué par des atomes d'oxygène ou de soufre, comme par exemple $CH_2C_6H_4OR$, $(CH_2)_xOR$, $(CH_2)_xSR'$, $(CH_2)_xCOOR'$, $(CH_2)_xSO_2R'$, $(CH_2)_xOCOR'$ avec x pouvant être égal à 0, 1 ou 2;
. soit par des amides (CONHR'', CONR''R'', CONHCHR''COOR', $CONHCHR''CONH_2$),des esters carboxyliques (COOR''), des dérivés halogénés (comme par exemple $CF_2R''$), des alcools (comme par exemple CHOHR'').

4. Edulcorants selon la revendication 3, caractérisés .en ce que:
- X est en position 4 et est choisi dans l'ensemble constitué par les groupes CN, $COOCH_3$, $COOC_2H_5$, $SO_2CH_3$, $COCH_3$;
- Y est choisi dans l'ensemble constitué par les groupes $COOCH_3$ ou $CH_3$;
- Z est choisi dans l'ensemble constitué par les groupes $CH_2C_6H_5$ (benzyl), $CH_2C_6H_{11}$ (cyclohexylméthyl), $C_6H_5$ (phényl), $C_6H_{11}$ (cyclohexyl), $C_5H_{11}$ (pentyl), iso-$C_5H_{11}$ (isopentyl), $CONHCH_2CH_2CH_3$ (propylamide), $CONHCH(CH_3)_2$ (isopropylamide), $CONHCH(C_3H_5)_2$ (dicyclopropylcarbinylamide), $CONHCH(C_3H_5)C(CH_3)_3$ (t-butylcyclopropylcarbinylamide).

5. Edulcorants selon la revendication 2, caractérisés en ce qu'ils consistent en des dérivés du N-(4-X-phénylcarbamoyl ou 4-X-phénylthiocarbamoyl)- L-aspartyl-L-phénylalanine méthyl ester de formule générale suivante:

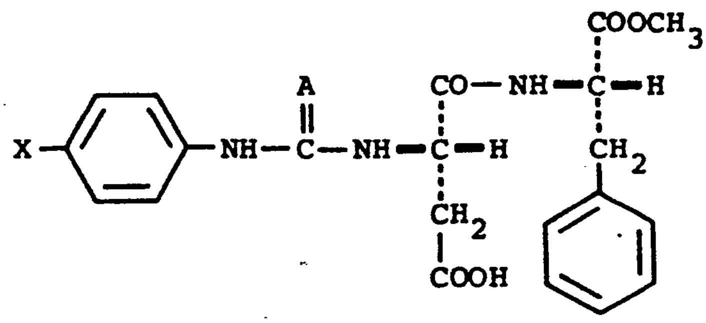

6. Edulcorants selon la revendication 2, caractérisés en ce qu'ils consistent en des dérivés de la N-(4-X-phénylcarbamoyl ou 4-X-phénylthiocarbamoyl)- L-aspartyl-L-1-(1-Z)éthanamine:

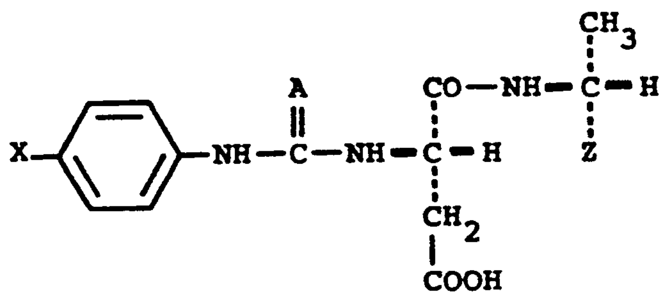

7. Edulcorants selon la revendication 2, caractérisés en ce qu'ils consistent en des dérivés du N-(4-X-phénylcarbamoyl ou 4-X-phénylthiocarbamoyl)-L-aspartyl-D-alaninamide:

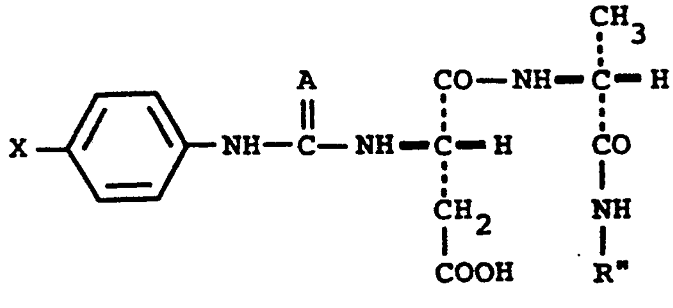

8. Procédé de préparation d'une substance consommable sucrée, caractérisé en ce qu'il consiste à ajouter à cette substance une quantité efficace d'un édulcorant selon l'une des revendications 2 à 7.

**Revendications** pour l'Etat contractant AT

1/ Procédé pour la préparation d'un composé de formule générale:

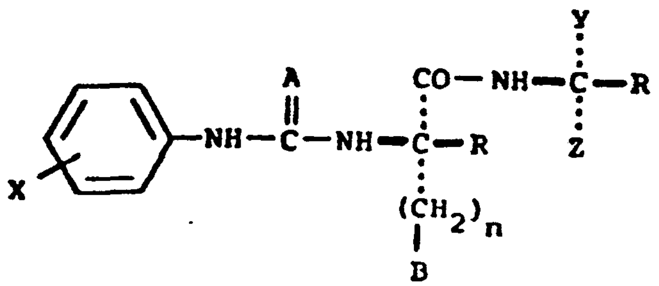

dans laquelle:

- X représente au moins un groupe donneur de doublets libres;
- A représente un atome d'oxygène ou de soufre ou un groupe imino ou méthylène;
- R represente un atome d'hydrogene ou un groupe alkyle de 1 à 4 atomes de carbone;
- n représente un nombre égal à zéro ou à un;
- B représente un groupe COOH ou COOM (M désignant un cation);
- Y représente soit un groupe donneur de doublets libres, soit un groupe alkyle R' de 1 à 4 atomes de carbone;
- Z représente:

. soit un groupe hydrophobe R", R" désignant un groupe alkyle, cycloalkyle, benzyle ou aryle ayant jusqu'à douze atomes de carbone;

. soit un groupe hydrophobe R", dans lequel 1 ou 2 atomes de carbone sont substitués par des atomes d'oxygène ou de soufre;

. soit un groupe hydrophobe R" fixé par un groupe amidique, ester, un carbone halogéné ou alcoolique,

caractérisé en ce qu'il consiste à condenser un premier composé de formule générale: $X\text{-}C_6H_4\text{-}N = C = A$ avec un second composé de formule générale:

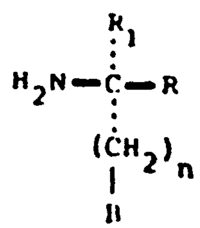

dans lesquelles X, A, R, n et B répondent aux définitions données ci-dessus et dans lesquelles $R_1$ désigne un radical choisi dans le groupe constitué par le radical -COOH et le radical

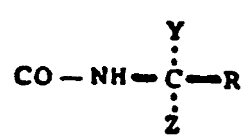

dans lequel Y et Z correspondent également aux définitions données ci-dessus.

2/ Procédé selon la revendication 1, caractérisé en ce que dans les composés utilisés:

- X est choisi dans l'ensemble constitué par les groupes CN, COOR, $SO_2R'$, COR', $NO_2$, halogènes, $SO_2NRR$, CONRR;
- A est choisi dans l'ensemble constitué par un atome d'oxygène ou de soufre;
- R est un atome d'hydrogène;
- n est égal à un;
- B est un groupe COOH ou COOM, M étant un cation choisi dans l'ensemble $Na^+$, $K^+$, $NH_4^+$, $Ca^{2+}$, $Mg^{2+}$ et $Li^+$;
- Y est choisi dans l'ensemble constitué soit par les groupes COOR', $CH_2OR$, $CHOHCH_3$, $CONHCH_2CH_2OH$, $CONHCHRCONH_2$, $CF_3$, $CCl_3$, soit un groupe R'; et
- Z est choisi dans l'ensemble constitué:

. soit par un groupe $CH_2C_6H_5$ (benzyl), $CH_2C_6H_{11}$ (cyclohexylméthyl), $C_6H_5$ (phényl), $C_6H_{11}$ (cyclohexyl), $C_5H_{11}$(pentyl), iso-$C_5H_{11}$(isopentyl), $C_4H_9$ (butyl), iso-$C_4H_9$ (isobutyl);

. soit par un de ces groupes substitué par des atomes d'oxygène ou de soufre, comme par exemple $CH_2C_6H_4OR$, $(CH_2)_xOR$, $(CH_2)_xSR'$,$(CH_2)_x$ COOR', $(CH_2)_xSO_2R'$ ou $(CH_2)_xOCOR'$ avec x pouvant être égal à 0, 1 ou 2;

. soit par des amides (CONHR'', CONR''R'', CONHCHR''COOR', CONHCHR''$CONH_2$), des esters carboxyliques (COOR''), des dérivés halogénés ou des alcools.

3/ Procédé selon la revendication 1 ou 2, caractérisé en ce que:

- X est en position 4 et est choisi dans l'ensemble constitué par les groupes CN, $COOCH_3$, $COOC_2H_5$ $SO_2CH_3$ et $COCH_3$;
- Y est choisi dans l'ensemble constitué par les groupes $COOCH_3$ et $CH_3$;
- Z est choisi dans l'ensemble constitué par les groupes $CH_2C_6H_5$ (benzyl), $CH_2C_6H_{11}$ (cyclohexylméthyl), $C_6H_5$ (phényl), $C_6H_{11}$ (cyclohexyl), $C_5H_{11}$(pentyl), iso-$C_5H_{11}$ (isopentyl), $CONHCH_2CH_2CH_3$ (propylamide), $CONHCH(CH_3)_2$ (isopropylamide), $CONHCH(C_3H_5)_2$ (dicyclopropylcarbinylamide) et CONHCH $(C_3H_5)C(CH_3)_3$ (t-butylcyclopropylcarbinylamide).

4/ Procédé selon la revendication 1, caractérisé en ce que la condensation est effectuée à température ambiante dans de l'eau, le premier composé étant préalablement dissous dans un solvant choisi dans le groupe constitué par le benzène, le chlorobenzène, le méthanol, l'éthanol.

5/ Procédé selon la revendication 1, caractérise:

- par le fait que $R_1$ est le groupe COOH,
- par le fait que B est choisi dans le groupe constitué par le groupe carboxyle libre et le groupe carboxyle libre préalablement protégé sous forme d'un ester benzylique ou tert-butylique,
- et en ce que le composé obtenu,de formule générale:

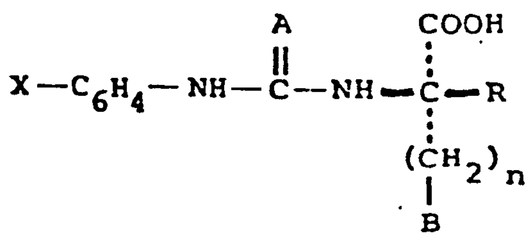

est ensuite mis en contact avec le composé de formule générale:

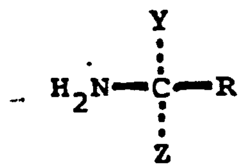

dans laquelle Y, Z et R désignent des radicaux précédemment définis.

6/ Procédé selon revendication 5, caractérisé en ce que la réaction est effectuée en présence d'un agent de déshydratation choisi dans le groupe constitué par les alcoxyacétylènes, les carbodiimides, les sels d'isoxazolium.

7/ Procédé selon revendication 5, caractérisé en ce que la réaction est effectuée enactivant le groupe carboxyle libre du composé intermédiaire obtenu, via un anhydride mixte formé in situ à partir d'un chloroformiate d'alkyle.

8/ Procédé selon revendication 5, dans lequel B est un groupe carboxyle libre et n est égal à un, caractérisé en ce qu'il consiste à déshydrater un diacide de formule générale:

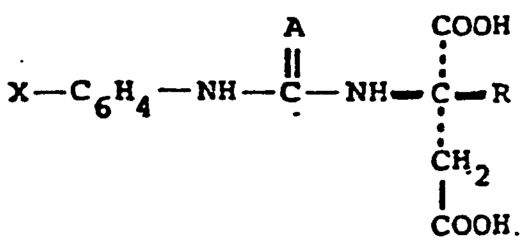

puis à mettre l'anhydride obtenu de formule générale:

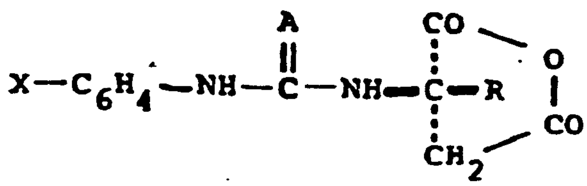

en présence d'un composé de formule générale:

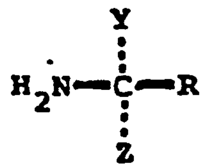

9/ Procédé selon la revendication 1, de préparation des dérivés du N-(4-X-phénylcarbamoyl ou 4-X-phénylthiocarbamoyl)-L-aspartyl-L-phénylalanine méthyl ester de formule générale suivante:

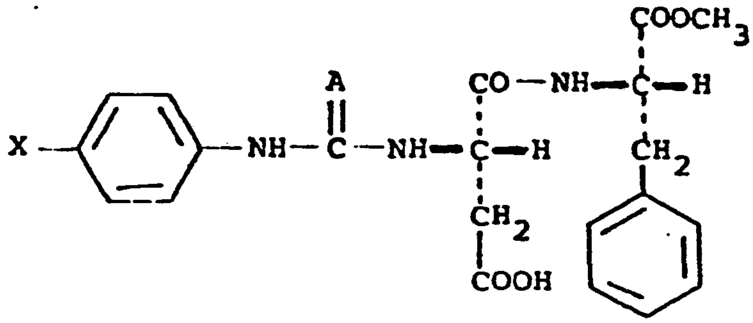

dans laquelle A et X ont la signification donnée précédemment.

10/ Procédé selon la revendication 1, de préparation des dérivés de la N-(4-X-phénylcarbamoyl ou 4-X-phénylthiocarbamoyl)-L-aspartyl-L-1-(1-Z) éthanamine de formule générale suivante:

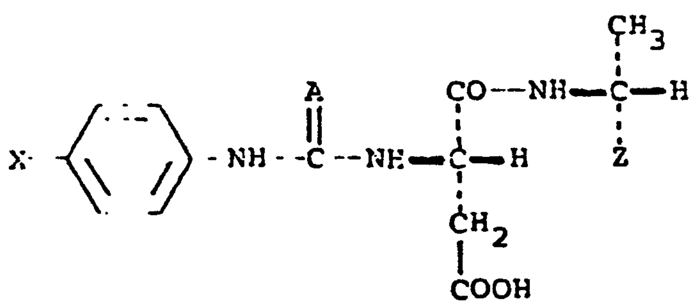

dans laquelle X, A et Z ont la signification donnée précédemment.

11/ Procédé selon la revendication 1, de préparation des dérivés du N-(4-X-phénylcarbamoyl ou 4-X-phénylthiocarbamoyl)-L-aspartyl-D-alaninamide de formule générale:

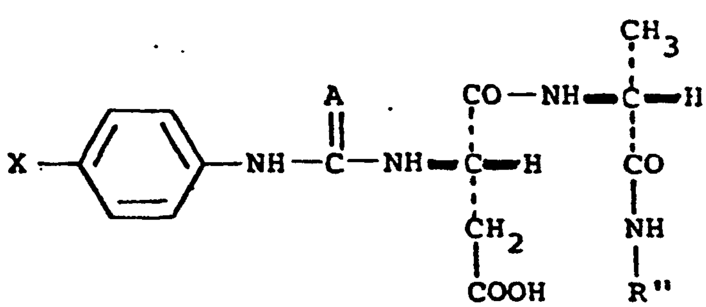

dans laquelle X, A et R'' ont la signification donnée précédemment.

12/ Procédé selon revendication 1, caractérisé en ce que le composé de formule générale:

$X-C_6H_4-N = C = A$

est condensé avec le L - aspartyl - L - phénylalanine méthyl ester.

13/ Procédé de préparation d'une substance consommable sucrée, caractérisé en ce qu'il consiste à ajouter à cette substance une quantité efficace d'un édulcorant selon la revendication 1.

**Patentansprüche** für die Vertragsstaaten: BE; CH; DE; FR; GB; IT; LI; LU; NL; SE

1. Chemische Verbindungen, dadurch gekenn- zeichnet, daß sie eine Verbindung der allgemeinen Formel

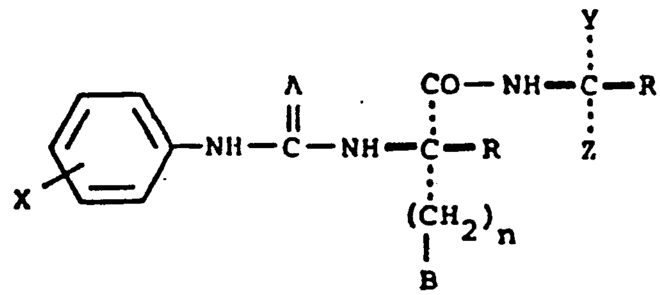

umfassen, in der
X mindestens eine Donatorgruppe freier Dubletten darstellt,
A ein Sauerstoff- oder Schwefelatom oder eine Imino- oder Methylengruppe darstellt,
R ein Wasserstoffatom oder einen Alkylrest mit 1 bis 4 Kohlenstoffatomen darstellt,
n die Zahl 0 oder 1 darstellt,
B die Gruppe -COOH oder -COOM bedeutet, wobei M ein Kation ist,
Y entweder eine Donatorgruppe freier Dubletten oder einen Alkylrest R' mit 1 bis 4 Kohlenstoffatomen darstellt und
Z entweder einen hydrophoben Rest R", wobei R" einen Alkyl-, Cycloalkyl-, Benzyl- oder Arylrest mit bis zu 12 Kohlenstoffatomen bezeichnet,
oder einen hydrophoben Rest R", in dem ein oder zwei Kohlenstoffatome durch Sauerstoff- oder Schwefelatome ersetzt sind,
oder einen hydrophoben Rest R" darstellt, der durch eine Amid-, Ester-, Halogenkohlenstoff- oder Alkoholgruppe bestimmt ist.

2. Synthetische Süßstoffe, dadurch gekennzeichnet, daß sie eine Verbindung der allgemeinen Formel

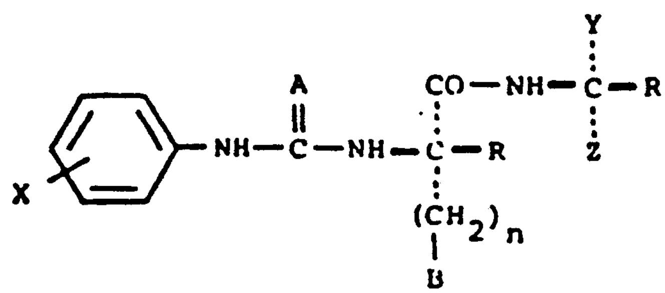

umfassen in der
X mindestens eine Donatorgruppe freier Dubletten darstellt,
A ein Sauerstoff- oder Schwefelatom oder eine Iminooder Methylengruppe darstellt,
R ein Wasserstoffatom oder einen Alkylrest mit 1 bis 4 Kohlenstoffatomen darstellt,
n die Zahl 0 oder 1 darstellt,
B die Gruppe -COOH oder -COOM bedeutet, wobei M ein Kation ist,
Y entweder eine Donatorgruppe freier Dubletten oder einen Alkylrest R' mit 1 bis 4 Kohlenstoffatomen darstellt und
Z entweder einen hydrophoben Rest R", wobei R" einen Alkyl-, Cycloalkyl-, Benzyl- oder Arylrest mit bis zu 12 Kohlenstoffatomen bezeichnet,
oder einen hydrophoben Rest R", in dem ein oder zwei Kohlenstoffatome durch Sauerstoff- oder Schwefelatome ersetzt sind,
oder einen hydrophoben Rest R" darstellt, der durch eine Amid-, Ester-, Halogenkohlenstoff- oder Alkoholgruppe bestimmt ist

3. Süßstoffe nach Anspruch 2, dadurch gekennzeichnet, daß in der Formel
X aus der Gruppe bestehend aus -CN, -COOR, $-SO_2R'$, -COR', $-NO_2$, Halogenen, $-SO_2NRR$ und -CONRR ausgewählt ist,
A aus der Gruppe bestehend aus Sauerstoff- oder Schwefelatom ausgewählt ist,

R ein Wasserstoffatom ist,
n die Zahl 1 ist,
B die Gruppe -COOH oder -COOM ist, wobei M ein aus der Gruppe bestehend aus $Na^+$, $K^+$, $NH_4^+$, $Ca^{2+}$, $Mg^{2+}$, $Li^+$ ausgewähltes Kation ist,
Y entweder aus der Gruppe bestehend aus -COOR', $-CH_2OR$, $-CHOHCH_3$, $-CONHCH_2CH_2OH$, $-CONHCHRCONH_2$, $-CF_3$, $-CCl_3$ oder aus der Gruppe R' ausgewählt ist, und
Z aus der Gruppe bestehend aus entweder-$CH_2C_6H_5$ (Benzyl), $-CH_2C_6H_{11}$ (Cyclohexylmethyl), $-C_6H_5$ (Phenyl), $-C_6H_{11}$ (Cyclohexyl), $-C_5H_{11}$ (Pentyl), iso-$C_5H_{11}$ (Isopentyl), $-C_4H_9$ (Butyl) und iso-$C_4H_9$ (Isobutyl), oder aus einer dieser durch Sauerstoff- oder Schwefelatome substituierten Gruppen, wie zum Beispiel $-CH_2C_6H_4OR$, $-(CH_2)_xOR$, $(CH_2)_xSR'$, $-(CH_2)_xCOOR'$, $-(CH_2)_xSO_2R'$, $-(CH_2)_xOCOR'$, wobei x = 0, 1 oder 2 sein kann, oder Amiden (-CONHR'', -CONR''R'', -CONHCHR''COOR', $-CONHCHR''CONH_2$), Carbonsäureestern (-COOR''), Halogenderivaten (wie zum Beispiel $-CF_2R''$), Alkoholen (wie zum Beispiel -CHOHR'') ausgewählt ist.

4. Süßstoffe nach Anspruch 3, dadurch gekennzeichnet, daß
X in der 4-Stellung steht und aus den Gruppen bestehend aus -CN, $-COOCH_3$, $-COOC_2H_5$, $-SO_2CH_3$, $-COCH_3$ ausgewählt ist,
Y aus der Gruppe bestehend aus $-COOCH_3$ oder $-CH_3$ ausgewählt ist und
Z aus der Gruppe bestehend aus $-CH_2C_6H_5$ (Benzyl), $-CH_2C_6H_{11}$ (Cyclohexylmethyl), $-C_6H_5$ (Phenyl), $-C_6H_{11}$ (Cyclohexyl), $-C_5H_{11}$ (Pentyl), iso-$C_5H_{11}$ (Isopentyl), $-CONHCH_2CH_2CH_3$ (N-Propylamid), $-CONHCH(CH_3)_2$ (N-Isopropylamide), $-CONHCH(C_3H_5)_2$ (N-Dicyclopropylcarbinylamid), $-CONHCH(C_3H_5)C(CH_3)_3$ (N-tert.-Butylcyclopropyl-carbinylamid) ausgewählt ist.

5. Süßstoffe nach Anspruch 2, dadurch gekennzeichnet, daß sie aus Derivaten des N-(4-X-Phenylcarbamoyl- oder 4-X-Phenylthiocarbamoyl)-L-aspartyl-L-phenylalanin-methyl-esters der nachstehenden allgemeinen Formel

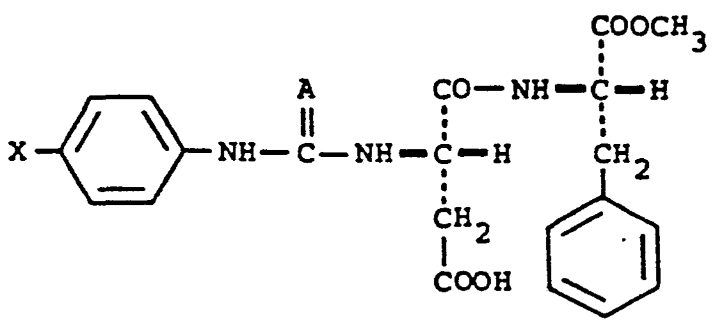

bestehen.

6. Süßstoffe nach Anspruch 2, dadurch gekennzeichnet, daß sie aus Derivaten des N-(4-X-phenylcarbamoyl- oder 4-X-Phenylthiocarbamoyl)-L-aspartyl-L-1-(1-Z)-ethanamins

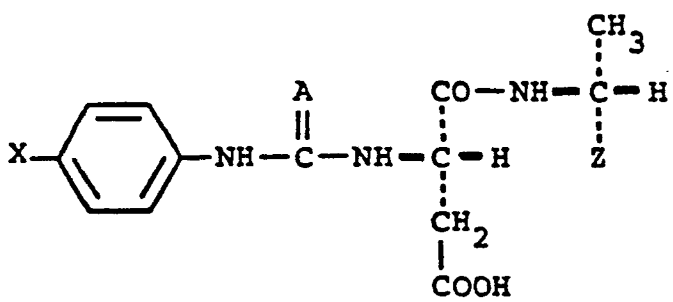

bestehen.

7. Süßstoffe nach Anspruch 2, dadurch gekennzeichnet, daß sie aus Derivaten des N-(4-X-Phenylcarbamoyl- oder 4-X-Phenylthiocarbamoyl)-L-aspartyl-D-alaninamids

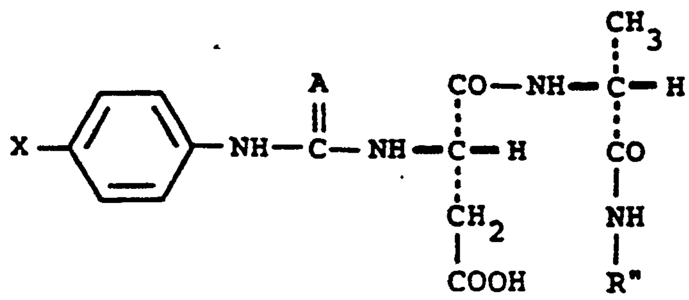

bestehen.

8. Verfahren zur Herstellung einer verzehrbaren süßenden Substanz, dadurch gekennzeichnet, daß es im Zufügen einer wirksamen Menge eines Süßstoffes nach einem der Ansprüche 2 bis 7 zu dieser Substanz besteht.

**Patentansprüche** für den Vertragsstaat: AT

1. Verfahren zur Herstellung einer Verbindung der allgemeinen Formel

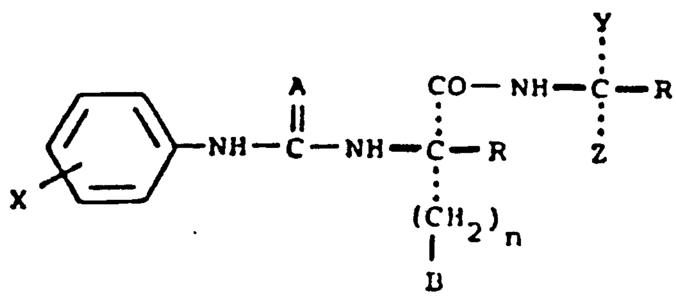

in der
X mindestens eine Donatorgruppe freier Dubletten darstellt,
A ein Sauerstoff- oder Schwefelatom oder eine Imino- oder Methylengruppe darstellt,
R ein Wasserstoffatom oder einen Alkylrest mit 1 bis 4 Kohlenstoffatomen darstellt.
n die Zahl 0 oder 1 darstellt,
B die Gruppe -COOH oder -COOM bedeutet, wobei M ein Kation ist,
Y entweder eine Donatorgruppe freier Dubletten oder einen Alkylrest R' mit 1 bis 4 Kohlenstoffatomen darstellt und
Z entweder einen hydrophoben Rest R'', wobei R'' einen Alkyl-, Cycloalkyl-, Benzyl- oder Arylrest mit bis zu 12 Kohlenstoffatomen bezeichnet,
oder einen hydrophoben Rest R'', in dem ein oder zwei Kohlenstoffatome durch Sauerstoff- oder Schwefelatome ersetzt sind,
oder einen hydrophoben Rest R'' darstellt, der durch eine Amid-, Ester-, Halogenkohlenstoff- oder Alkoholgruppe bestimmt ist, dadurch gekennzeichnet, daß es im Kondensieren einer ersten Verbindung der allgemeinen Formel

$X - C_6H_4 - N = C = A$

mit einer zweiten Verbindung der allgemeinen Formel

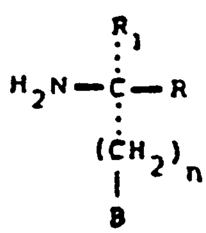

in der X, A, R, n und B die oben angegebenen Bedeutungen haben und $R_1$ einen aus der Gruppe bestehend aus dem Rest -COOH und dem Rest

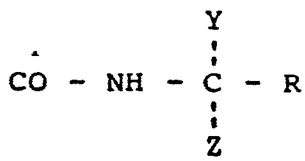

wobei Y und Z in gleicher Weise die oben angegebenen Bedeutungen haben, besteht.

2. Verfahren nach Anspruch 1, dadurch gekennzeichnet, daß bei den verwendeten Verbindungen

X aus der Gruppe bestehend aus -CN, -COOR, $-SO_2R'$, -COR' $-NO_2$, Halogenen, $-SO_2NRR$, -CONRR ausgewählt ist,

A aus der Gruppe bestehend aus Sauerstoff- oder Schwefelatom ausgewählt ist,

R ein Wasserstoffatom ist,

n die Zahl 1 ist,

B die Gruppe -COOH oder -COOM ist, wobei M ein aus der Gruppe bestehend aus $Na^+$, $K^+$, $NH_4^+$, $Ca^{2+}$, $Mg^{2+}$ und $Li^+$ ausgewähltes Kation ist,

Y entweder aus der Gruppe bestehend aus -COOR', $-CH_2OR$, $-CHOHCH_3$, $-CONHCH_2CH_2OH$, $-CONHCHRCONH_2$, $-CF_3$, $-CCl_3$ oder aus der Gruppe R' ausgewählt ist, und

Z aus der Gruppe bestehend aus entweder-$CH_2C_6H_5$ (Benzyl), $-CH_2C_6H_{11}$ (Cyclohexylmethyl), $-C_6H_5$ (phenyl), $-C_6H_{11}$ (Cyclohexyl), $-C_5H_{11}$ (Pentyl), iso-$C_5H_{11}$ (Isopentyl), $-C_4H_9$ (Butyl) und iso-$C_4H_9$ (Isobutyl),

oder aus einer dieser durch Sauerstoff- oder Schwefelatome substituierten Gruppen, wie zum Beispiel $-CH_2C_6H_4OR$, $-(CH_2)_xOR$, $-(CH_2)_xSR'$, $-(CH_2)_xCOOR'$, $-(CH_2)_xSO_2R'$oder $-(CH_2)_xOCOR'$, wobei x = 0, 1 oder 2 sein kann,

oder Amiden (-CONHR", -CONR"R", -CONHCHR"COOR', $-CONHCHR"CONH_2$), Carbonsäureestern (-COOR"), Halogenderivaten oder Alkoholen ausgewählt ist.

3. Verfahren nach Anspruch 1 oder 2, dadurch gekennzeichnet, daß

X in der 4-Stellung steht und aus den Gruppen bestehend aus -CN,$-COOCH_3$,$-COOC_2H_5$, $-SO_2CH_3$ und $-COCH_3$ ausgewählt ist,

Y aus der Gruppe bestehend aus $-COOCH_3$ oder $-CH_3$ ausgewählt ist und

Z aus der Gruppe bestehend aus $-CH_2C_6H_5$ (Benzyl), $-CH_2C_6H_{11}$ (Cyclohexylmethyl), $-C_6H_5$ (phenyl), $-C_6H_{11}$ (Cyclohexyl), $-C_5H_{11}$ (pentyl), iso-$C_5H_{11}$ (Isopentyl), $-CONHCH_2CH_2CH_3$ (N-Propylamid), $-CONHCH(CH_3)_2$ (N-Isopropylamide), $-CONHCH(C_3H_5)_2$ (N-Dicyclopropylcarbinylamid) und $-CONHCH(C_3H_5)C(CH_3)_3$ (N-tert.-Butylcyclopropyl-carbinylamid) ausgewählt ist.

4. Verfahren nach Anspruch 1, dadurch gekennzeichnet, daß die Kondensation bei gewöhnlicher Temperatur in Wasser durchgeführt wird, wobei die erste Verbindung zuvor in einem Lösungsmittel, das aus der Gruppe bestehend aus Benzol Chlorbenzol, Methanol, Ethanol ausgewählt ist, gelöst wird.

5. Verfahren nach Anspruch 1, dadurch gekennzeichnet, daß für den Fall, daß $R_1$ die Gruppe -COOH ist, und für den Fall, daß B aus der Gruppe bestehend aus der freien Carboxylgruppe und der freien; zuvor unter Bildung eines Benzyloder tert.-Butylesters geschützten Carboxylgruppe ausgewählt ist, die erhaltene Verbindung der allgemeinen Formel

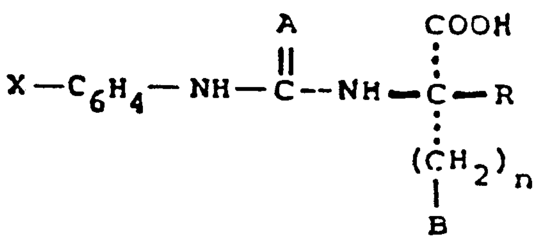

danach mit der Verbindung der allgemeinen Formel

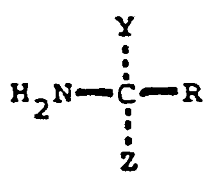

in der Y, Z und R der vorstehend definierten Reste bezeichnen, in Berührung gebracht wird.

6. Verfahren nach Anspruch 5, dadurch gekennzeichnet, daß die Umsetzung in Gegenwart eines Dehydratisierungsmittels, das aus der Gruppe bestehend aus Alkoxyacetylenen, Carbodiimiden und Isoxazoliumsalzen ausgewählt ist, durchgeführt wird.

7. Verfahren nach Anspruch 5, dadurch gekennzeichnet, daß die Umsetzung durch Aktivierung der freien

Carboxylgruppe der erhaltenen Zwischenverbindung über ein gemischtes Anhydrid, das in situ von einem Chlorameisensäure-ethylester gebildet ist, durchgeführt wird.

8. Verfahren nach Anspruch 5, wobei B eine freie Carboxylgruppe bedeutet und n = 1 ist, dadurch gekennzeichnet, daß es in einer Dehydratisierung einer Disäure der allgemeinen Formel

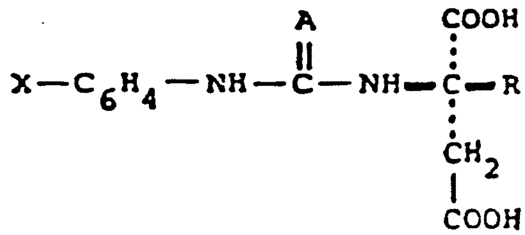

und danach in einem Umsetzen des erhaltenen Anhydrids der allgemeinen Formel

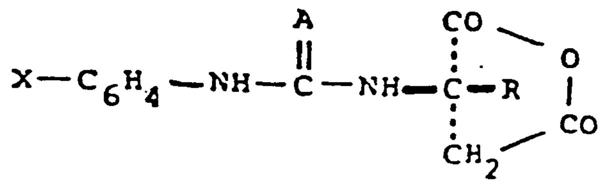

in Gegenwart einer Verbindung der allgemeinen Formel

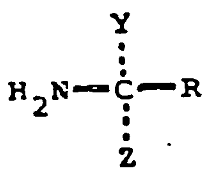

besteht.

9. Verfahren nach Anspruch 1 zur Herstellung von Derivaten des N-(4-X-Phenylcarbamoyl- oder 4-X-Phenylthiocarbamoyl)-Laspartyl-L-phenylalanin-methylesters der nachstehenden allgemeinen Formel

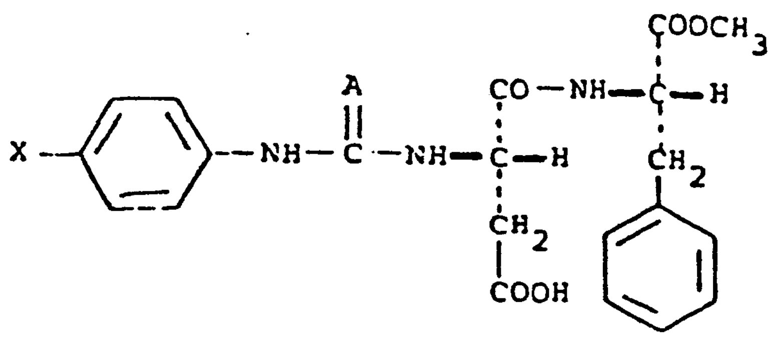

in der A und X die vorstehend angegebenen Bedeutungen haben.

10. Verfahren nach Anspruch 1 zur Herstellung von Derivaten des N-(4-X-Phenylcarbamoyl- oder 4-X-Phenylthiocarbamoyl)-L-aspartyl-L-1-(1-Z)-ethanamins der nachstehenden allgemeinen Formel

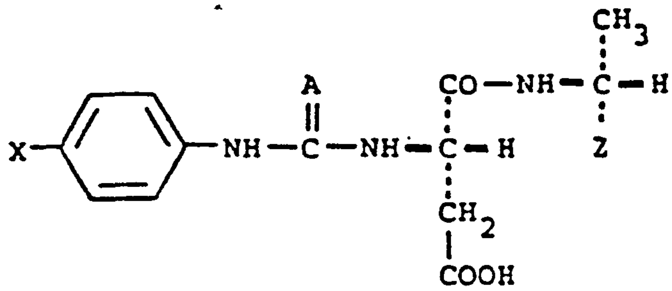

in der X, A, und Z die vorstehend angegebenen Bedeutungen haben.

11. Verfahren nach Anspruch 1 zur Herstellung von Derivaten des N-(4-X-Phenylcarbamoyl- oder 4-X-Phenylthiocarbamoyl)-L-aspartyl-D-alaninamids der allgemeinen Formel

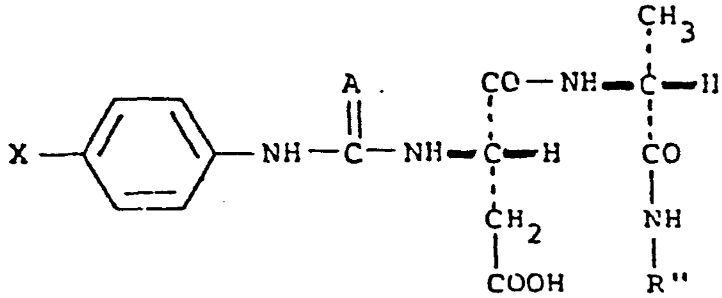

in der X, A und R'' die vorstehend angegebenen Bedeutungen haben.

12. Verfahren nach Anspruch 1, dadurch gekennzeichnet, daß die Verbindung der allgemeinen Formel $X\text{-}C_6H_4\text{-}N = C = A$ mit L-Aspartyl-L-phenylalanin-methylester kondensiert wird.

13. Verfahren zur Herstellung einer verzehrbaren süßenden Substanz, dadurch gekennzeichnet, daß es im Zufügen einer wirksamen Menge eines Süßstoffes nach Anspruch 1 zu dieser Substanz besteht.

**Claims** for the contracting States: BE, CH, DE, FR, GB, IT, LI, LU, NL, SE

1/ Chemical compounds of formula:

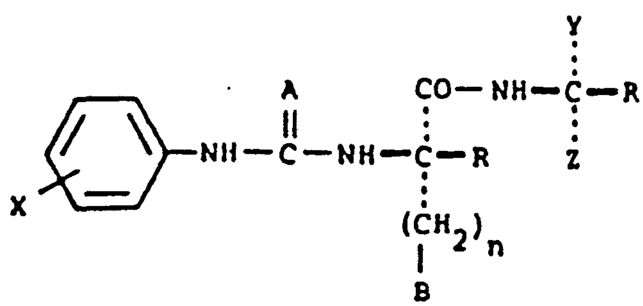

wherein:
- X is a lone-electron pair donor group;
- A is an oxygen or a sulfur atom, or an imino or a methylene group;
- R is a hydrogen atom or an alkyl group with 1 to 4 carbon atoms;
- n is zero or one;
- B is COOH or COOM group (M being a cation);
- Y is either a lone-electron pair donor group, or an alkyl group R' with 1 to 4 carbon atoms;
- Z is:

. either a hydrophobic group R'', R'' being an alkyl, cycloalkyl, benzyl or aryl group having up to twelve carbon atoms,

. or a hydrophobic group R'' in which one or two carbon atoms are substituted by oxygen or sulfur atoms,

. or a hydrophobic group R'' fixed by an amidic or an ester group, or a halogenated or alcoholic carbon.

2/ Synthetic sweeteners of formula

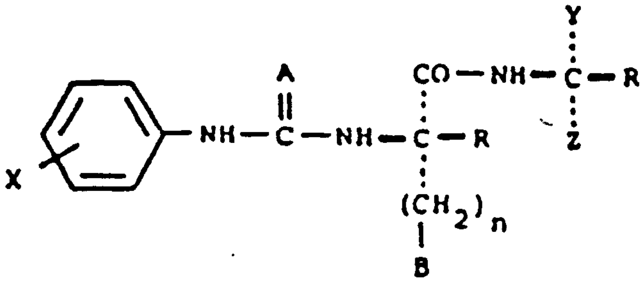

wherein:

- X is a lone-electron pair donor group;
- A is an oxygen or a sulfur atom, or an imino or a methylene group;
- R is a hydrogen atom or an alkyl group with 1 to 4 carbon atoms;
- n is zero or one;
- B is COOH or COOM group (M being a cation);
- Y is either a lone-electron pair donor group, or an alkyl group R' with 1 to 4 carbon atoms;
- Z is:

. either a hydrophobic group R'', R'' being an alkyl, cycloalkyl, benzyl or aryl group having up to twelve carbon atoms,

. or a hydrophobic group R'' in which one or two carbon atoms are substituted by oxygen or sulfur atoms,

. or a hydrophobic group R'' fixed by an amidic or an ester group, or a halogenated or an alcoholic carbon.

3/ Sweeteners according to claim 2, wherein:

X is CN, COOR, $SO_2R'$, COR', $NO_2$, a halogen atom, $SO_2NRR$, CONRR;

- A is an oxygen or a sulfur atom;
- R is a hydrogen atom;
- n is one;
- B is a COOH or COOM group wherein M is a cation selected from the group constituted by $Na^+$, $K^+$, $NH_4^+$, $Ca^{2+}$, $Mg^{2+}$, $Li^+$;
- Y is selected from the group constituted by COOR', $CH_2OR$, $CHOHCH_3$, $CONHCH_2CH_2OH$, $CONHCHRCONH_2$, $CF_3$, $CCl_3$ groups, or by a R' group;
- Z is selected from the group constituted;

. either by a group $CH_2C_6H_5$ (benzyl), $CH_2C_6H_{11}$ (cyclohexylmethyl), $C_6H_5$ (phenyl), $C_6H_{11}$ (cyclohexyl), $C_5H_{11}$ (pentyl), iso-$C_5H_{11}$ (isopentyl), $C_4H_9$ (butyl), iso-$C_4H_9$ (isobutyl),

. either by one of these groups substituted by an oxygen or a sulfur atom, such as $CH_2C_6H_4OR$, $(CH_2)_xOR$, $(CH_2)_xSR'$, $(CH_2)_xCOOR'$, $(CH_2)_xSO_2R'$, $(CH_2)_xOCOR'$ with x being equal to 0, 1 or 2,

. or by amides (CONHR'', CONR''R'', CONHCHR''COOR', CONHCHR''CONH2), carboxylic esters (COOR''), halogenated derivatives (such as $CF_2R''$), alcohols (such as CHOHR'').

4/ Sweeteners according to claim 3, wherein:

- X is in 4 position and is selected from the group constituted by the CN, $COOCH_3$, $COOC_2H_5$, $SO_2CH_3$, $COCH_3$ groups;
- Y is selected from the group constituted by the groups $COOCH_3$ or $CH_3$;
- Z is selected from the group constituted by the groups $CH_2C_6H_5$ (benzyl), $CH_2C_6H_{11}$ (cyclohexylmethyl), $C_6H_5$ (phenyl), $C_6H_{11}$ (cyclohexyl), $C_5H_{11}$ (pentyl), iso-$C_5H_{11}$ (isopentyl), $CONHCH_2CH_2CH_3$ (propylamide), $CONHCH(CH_3)_2$ (isopropylamide), $CONHCH(C_3H_5)_2$ (dicyclopropylcarbinylamide), $CONHCH(C_3H_5)C(CH_3)_3$ (t-butylcyclopropylcarbinylamide).

5/ Sweeteners according to claim 2, consisting in a derivative of N-(4-X-phenylcarbamoyl or 4-X-phenylthiocarbamoyl)-L-aspartyl-L-phenylalanine methyl ester of the following general formula:

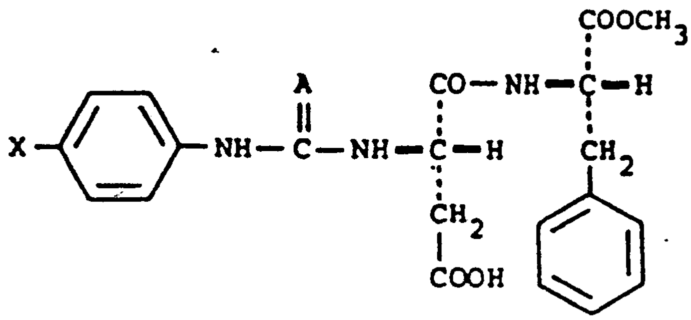

6/ Sweeteners according to claim 2, consisting in a derivative of N-(4-X-phenylcarbamoyl or 4-X-phenylthiocarbamoyl)-L-aspartyl-L-1-(1-Z)ethanamine:

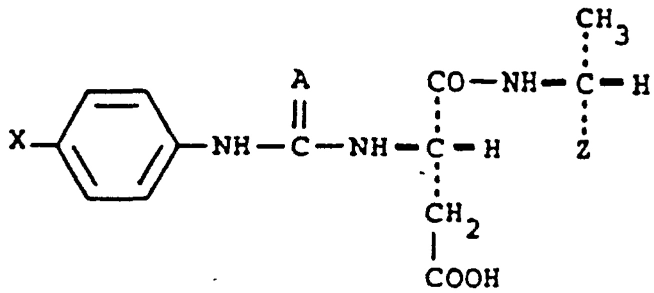

7/ Sweeteners according to claim 2, consisting in a derivative of N-(4-X-phenylcarbamoyl or 4-X-phenyl thiocarbamoyl)-L-aspartyl-D-alaninamide:

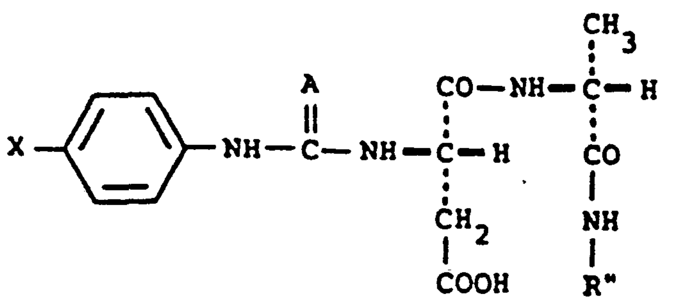

8/ A process to prepare a sweetened consumable food comprising, adding to this food, an efficient quantity of a sweetener according to any claims 2 to 7.

**Claims** for the contracting State: AT

1/ A process to prepare a compound of formula:

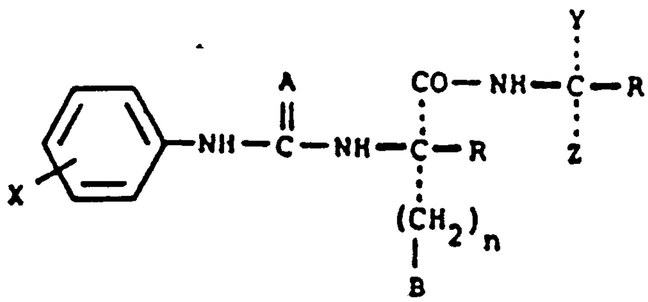

wherein:
- X is a lone-electron pair donor group;
- A is an oxygen or a sulfur atom, or an imino or a methylene group;
- R is a hydrogen atom or an alkyl group with 1 to 4 carbon atoms;
- n is zero or one;
- B is COOH or COOM group (M being a cation);
- Y is either a lone-electron pair donor group, or an alkyl group R' with 1 to 4 carbon atoms;
- Z is:

. either a hydrophobic group R'', R'' being an alkyl, cycloalkyl, benzyl or aryl group having up to twelve carbon atoms,

. or a hydrophobic group R'' in which one or two carbon atoms are substituted by oxygen or sulfur atoms,

. or a hydrophobic group R'' fixed by an amidic or an ester group, or a halogenated or alcoholic carbon,

consisting to condensate a first compound of formula:

$X\text{-}C_6H_4\text{-}N = C = A$

with a second compound of formula:

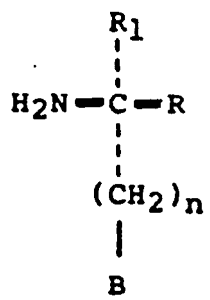

with the groups X, A, R, n and B as defined hereinabove and within the group $R_1$ represents either a COOH group or a group:

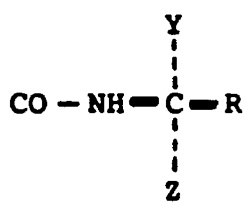

with Y and Z as defined hereinabove.

2/ Process according to claim 1, wherein:
- X is CN, COOR, $SO_2R'$, COR',.$NO_2$, a halogen atom, $SO_2NRR$, CONRR;
- A is an oxygen or a sulfur atom;
- R is a hydrogen atom;
- n is one;
- B is a COOH or COOM group wherein M is a cation selected from the group constituted by $Na^+$, $K^+$, $NH_4^+$, $Ca^{2+}$, $Mg^{2+}$, $Li^+$;
- Y is selected from the group constituted by COOR', $CH_2OR$, $CHOHCH_3$, $CONHCH_2CH_2OH$, $CONHCHRCONH_2$, $CF_3$, $CCl_3$ groups, or by a R' group;
- Z is selected from the group constituted;

. either by a group $CH_2C_6H_5$ (benzyl), $CH_2C_6H_{11}$ (cyclohexylmethyl), $C_6H_5$ (phenyl), $C_6H_{11}$ (cyclohexyl), $C_5H_{11}$ (pentyl), iso-$C_5H_{11}$ (isopentyl), $C_4H_9$ (butyl), iso-$C_4H_9$ (isobutyl),

. either by one of these groups substituted by an oxygen or a sulfur atom, such as $CH_2C_6H_4OR$, $(CH_2)_xOR$,

$(CH_2)_xSR'$, $(CH_2)_xCOOR'$, $(CH_2)_x SO_2R'$, $(CH_2)_xOCOR'$ with x being equal to 0, 1 or 2,

. or by amides (CONHR", CONR"R", CONHCHR"COOR', $CONHCHR"CONH_2$), carboxylic esters (COOR"), halogenated derivatives (such as $CF_2R"$), alcohols (such as CHOHR").

3/ Process according to claims 1 to 2, wherein:

- X is in 4 position and is selected from the group constituted by the CN, $COOCH_3$, $COOC_2H_5$, $SO_2CH_3$, $COCH_3$ groups;

- Y is selected from the group constituted by the groups $COOCH_3$ or $CH_3$;

- Z is selected from the group constituted by the groups $CH_2C_6H_5$ (benzyl), $CH_2C_6H_{11}$ (cyclohexylmethyl), $C_6H_5$ (phenyl), $C_6H_{11}$ (cyclohexyl), $C_5H_{11}$ (pentyl), iso-$C_5H_{11}$ (isopentyl), $CONHCH_2CH_2CH_3$ (propylamide), $CONHCH(CH_3)_2$ (isopropylamide), $CONHCH(C_3H_5)_2$ (dicyclopropylcarbinylamide), $CONHCH(C_3H_5)C(CH_3)_3$ (t-butylcyclopropylcarbinylamide).

4/ Process according to claim 1, wherein the condensation is carried out in water at room temperature, the first compound being previously dissolved in a solvent selected from the group comprising benzene, chlorobenzene, methanol, ethanol.

5/ Process according to claim 1, wherein:

- $R_1$ is the group COOH,

- B is selected from the group constituted by the free carboxyl group and the previously protected carboxyl group in the form of a benzyl or t-butyl ester;

- and wherein the obtained compound, of general formula:

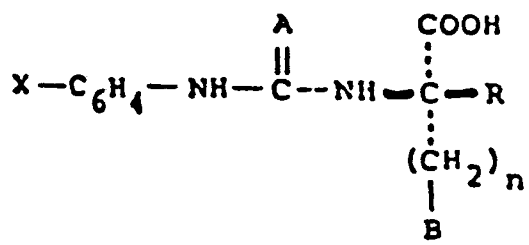

is therefore brought into contact with the compound of general formula:

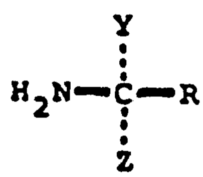

in which Y, Z and R are such as defined hereinabove.

6/ Process according to claim 5, wherein the reaction is effected in the presence of an.appropriate dehydrating agent selected from the group constituted by alkoxyacetylenes, carbodiimides, isoxazolium sâlts.

7/ Process according to claim 5, wherein the reaction is carried out by activating the free carboxyl group of the intermediate compound obtained via a mixed anhydride formed in situ from an alkyl chloroformate.

8/ Process according to claim 5, wherein B is a free carboxyl group and n is one, consisting to dehydrate a diacid of general formula:

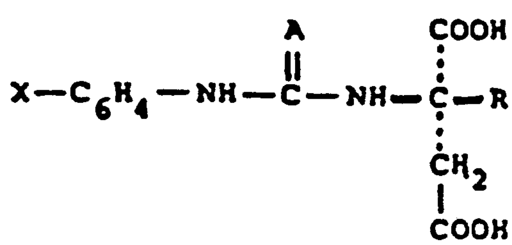

then brought the anhydride obtained of general formula:

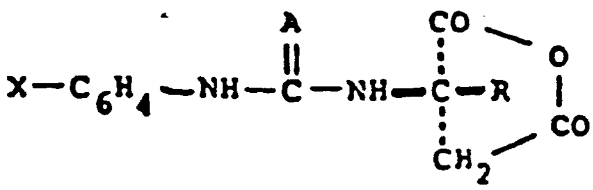

into contact with compound of general formula:

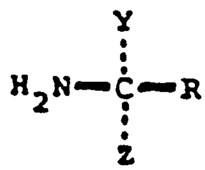

9/ Process according to claim 1 for preparation of derivatives of N-(4-X-phenylcarbamoyl or 4-X-phenylthiocarbamoyl)-L-aspartyl-L-phenylalanine methyl ester of general formula:

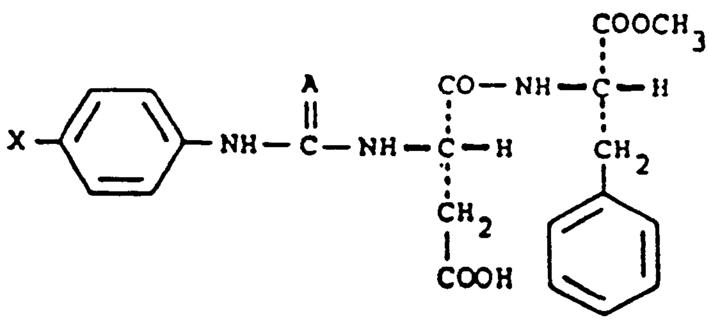

wherein A and X as defined hereinabove.

10/ Process according to claim 1 for preparation of derivatives of N-(4-X-phenylcarbamoyl or 4-X-phenylthiocarbamoyl)-L-aspartyl-L-1-(1-Z)ethanamine of general formula:

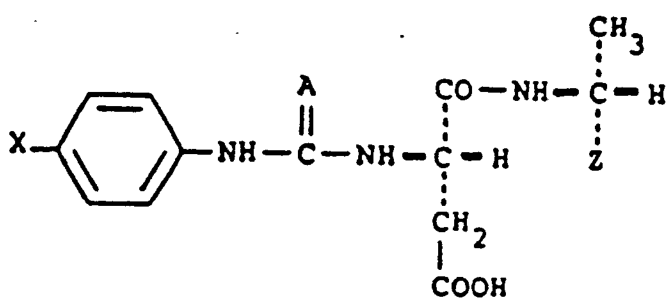

wherein X, A and Z as defined hereinabove.

11/ Process according to claim 1 for preparation of derivatives of N-(4-X-phenylcarbamoyl or 4-X-phenylthiocarbamoyl)-L-aspartyl-D-alaninamide of general formula:

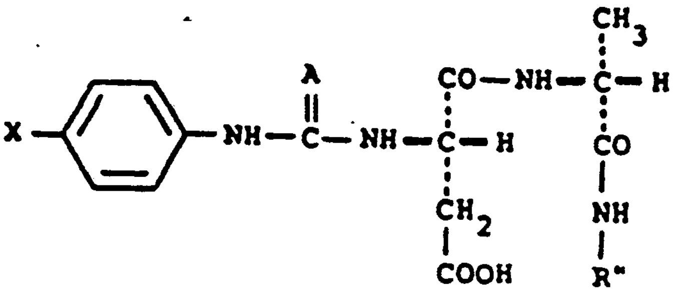

wherein X, A and R'' as defined hereinabove.

12/ Process according to claim 1, wherein the compound of general formula:

$X\text{-}C_6H_4\text{-}N{=}C{=}A$

is condensated with the L-aspartyl-L-phenylalanine methyl ester.

13/ Process for preparation a sweetened consumable food, consisting to add to this food an efficient quantity of a sweetener according to claim 1.